# EUROPEAN PATENT SPECIFICATION

(11) **EP 2 508 120 B1**
(45) Date of publication and mention of the grant of the patent: **02.01.2019**
(21) Application number: 12154569.3
(22) Date of filing: 08.03.2007
(51) Int. Cl.: A61B 1/018, A61B 1/005, A61B 1/00

(54) **Catheter assembly**
Katheterverbindung
Arrangement pour un cathéter

(30) Priority: 23.03.2006 US 388247
(43) Date of publication of application: 10.10.2012
(62) Divisional of application: 07758182.5
(73) Proprietor: Boston Scientific Limited, Hamilton HM11 (BM)
(72) Inventor: Freed, David I., Westborough MA 01581 (US); Golden, John B., Norton, MA 02766 (US); Chu, Michael S. H., Brookline MA 02446 (US); Carrillo, Oscar R., Jr., Attleboro, Massachusetts 02703 (US); Chin, Yem, Burlington MA 01803 (US); Adams, Mark, L., Sandy, UT 84092 (US); Morris, Benjamin, E., Jeffersonville, Indiana 47130 (US); Wells, Brian, Keith, LaGrange, KY 40031 (US); Hall, Todd, A., Goshen, KY 40026 (US); Furnish, Gregory, R., Louisville, KY 40206 (US); Abramov, Vasiliy, P., Louisville, KY 40205 (US); Mers-Kelly, William, C., Crestwood, KY 40014 (US)
(74) Representative: Vossius & Partner Patentanwälte Rechtsanwälte mbB

(56) References cited:
- EP-A1- 1 502 537
- EP-A1- 1 568 305
- EP-A1- 1 842 499
- EP-A2- 1 302 151
- US-A1- 2004 015 050

## Description

### FIELD OF THE INVENTION

The invention relates to a catheter assembly for mounting a catheter to an endoscopic device.

### BACKGROUND

A challenge in the exploration and treatment of internal areas of the human anatomy has been adequately visualizing the area of concern. Visualization can be especially troublesome in minimally invasive procedures in which small diameter, elongate instruments, such as catheters and endoscopes, are navigated through natural passageways of a patient to an area of concern either in the passageway or in an organ reachable through the passageway.

Detailed information regarding the anatomy can be discerned from direct viewing of the anatomy provided through one or more of the elongate instruments used in the procedure. Various types of endoscopes configured for use in various passageways of the body such as the esophagus, rectum, or bronchus can be equipped with direct viewing capability through the use of optical fibers extending through the length of the scope, or with digital sensors, such as CCD or CMOS. However, because endoscopes also provide a working channel through which other medical instruments must pass, optional lighting bundles and components to provide steering capability at its distal end, the scope is typically of a relatively large diameter, e.g., 5 mm or greater. This large diameter limits the use of the endoscope to relatively large body lumens and prohibits their use in smaller ducts and organs that branch from a large body lumen, such as the biliary tree.

Typically, when examining small passageways such as the bile duct or pancreatic duct, the endoscope is used to get close to a smaller passageway or region of concern, and another instrument, such as a catheter, is then extended through the working channel of the endoscope and into the smaller passageway. Although the endoscope provides direct visualization of the large body passageway and entrance to adjoining ducts and lumens, after the smaller catheter has been extended from the endoscope into the smaller duct or lumen, direct visualization has heretofore been limited, and the physician usually relies on radiographical means to visualize the area of concern or probes blindly.

The document EP1502537 by Olympus Corp discloses a catheter assembly system with a slide bar mechanism for mounting a catheter to an endoscope.

### SUMMARY

The present invention is defined by the features of independent claim 1. Further preferred embodiments of the invention are defined in the dependent claims. Moreover, further illustrative embodiments, which are useful for the understanding of the present invention, are discussed in the following.

This summary is provided to introduce a selection of concepts in a simplified form that are further described below in the Detailed Description. This summary is not intended to identify key features of the claimed subject matter, nor is it intended to be used as an aid in determining the scope of the claimed subject matter.

In one illustrative example a medical device comprising a control handle, an insertion tube functionally connected to the control handle, the insertion tube including a channel extending lengthwise therethrough, and a user-actuable control feature for controlling at least one function of the medical device. The control feature includes a stylet having a proximal end associated with the control handle and a free distal end associated with the insertion tube, the stylet being movably carried by the control handle along at least a portion of the channel, wherein the stylet is capable of deflecting a distal end of the insertion tube upon user input occurring at the control handle.

In another illustrative example, a medical system, comprising a catheter having a proximal end and a distal end, the catheter including at least one steering wire, and a control handle that includes a first opening in communication with a second opening, an attachment structure configured for selectively associating the control handle with a port of an associated endoscopic device, and a steering input device connected to the at least one steering wire for deflecting the distal end of the catheter. The proximal end of the catheter is functionally connected to the control handle and the distal end of the catheter is inserted into the first opening of the control handle and exits the second opening of the control handle for insertion into the port of the associated endoscopic device when associated therewith.

In another illustrative example, a catheter assembly comprising a catheter having a proximal end and a distal end; a steering wire attached to the distal end of the catheter and being axially movable relative to the catheter to cause the distal end of the catheter to deflect in at least one direction; a handle housing functionally connected to the proximal end of the catheter at a connection interface, the proximal end of the at least one steering wire passing into the handle housing; a first knob rotationally supported by the handle housing, the rotational axis of the knob being
substantially parallel with the central axis of the catheter at its connection interface to the handle housing; and a transmission connecting the at least one knob with the steering wire.

In another illustrative example, an apparatus is provided, which comprises a control handle; an insertion tube having proximal and distal sections, the proximal section being functionally attached to the handle and the distal section being opposite the proximal section and being deflectable relative to the proximal section; a steering wire attached to the distal section of the insertion tube and being axially movable relative to the insertion tube to cause the distal section of the insertion tube to deflect in at least one direction; an input device movably carried by the handle and adapted to control deflection of the distal section, the input device being movable by a user relative to the handle during operation of the apparatus; and a transmission connected at a first portion to the steering wire and connected at a second portion to the input device. The transmission is configured to transmit movement of the input device to the steering wire to cause axial movement of the steering wire for deflecting the distal end of the insertion tube, the transmission is further configured for providing a distance multiplying effect in transmitting the movement of the input device to axial movement of the steering wire for deflection of the distal section.

### DESCRIPTION OF THE DRAWINGS

The foregoing aspects and many of the attendant advantages of this invention will become more readily appreciated by reference to the following detailed description, when taken in conjunction with the accompanying drawings, wherein:
FIGURE 1 is a front elevational view of one representative embodiment of an *in vivo* visualization system;
FIGURE 2 is a lateral cross-sectional view of an insertion tube of an endoscope shown in FIGURE 1;
FIGURE 3 is a perspective view of one embodiment of a catheter assembly;
FIGURE 4 is a perspective view of the catheter assembly shown in FIGURE 3 with one housing half removed;
FIGURES 5A-5C illustrate cross-sectional views of suitable embodiments of a catheter;
FIGURE 6A is a partial view of one suitable embodiment of a catheter body;
FIGURE 6B is a partial view of one suitable embodiment of a catheter formed by taking the catheter body of FIGURE 6A and encasing said catheter body with a reinforcement sheath;
FIGURE 6C is a partial view of one suitable embodiment of a catheter formed by taking the catheter of FIGURE 6B and encasing said catheter with an outer sleeve;
FIGURE 7 is a cross-sectional view of the catheter taken along plane 7-7 shown in FIGURE 6C;
FIGURES 8A-8C are cross-sectional views of suitable embodiments of a catheter;
FIGURES 9A-9C are cross-sectional views of suitable embodiments of a catheter;
FIGURE 10 is a partial perspective view of a catheter handle with the control knobs removed to illustrate a lock lever;
FIGURE 11 is a partial cross-sectional view of a catheter handle showing a suitable embodiment of an irrigation port connected to irrigation lumens of the catheter;
FIGURE 12 is a partial cross-section view of the catheter handle showing the steering mechanism and the optional locking mechanism;
FIGURE 13A is a front exploded perspective view of components of the locking mechanism of FIGURE 12;
FIGURE 13B is a rear exploded perspective view of components of the locking mechanism of FIGURE 12;
FIGURE 14 is a partial perspective view of the catheter handle of FIGURE 11 illustrating a suitable embodiment of an endoscope attachment device;
FIGURE 15 is a cross-sectional view of one embodiment of a Y connector when assembled with a catheter;
FIGURE 16A is a front perspective view of another embodiment of a catheter assembly;
FIGURE 16B is a rear perspective view of the catheter assembly of FIGURE 16A;
FIGURE 16C is an end view of one embodiment of a catheter suitable for use with the catheter assembly of FIGURE 16A;
FIGURE 17A is one embodiment of a stylet suitable for use with the catheter assembly of FIGURES 16A-16C, wherein the stylet includes a preformed distal end region;
FIGURE 17B illustrates the stylet of FIGURE 17A inserted into the proximal section of a catheter;
FIGURE 17C illustrates the stylet of FIGURE 17A inserted into the distal section of the catheter shown in FIGURE 17B;
FIGURE 18A is another embodiment of a stylet suitable for use with the catheter assembly of FIGURES 16A-16C, the stylet being illustrated in its straight configuration;
FIGURE 18B illustrates the stylet of FIGURE 18A in its bent distal end region configuration;
FIGURE 18C illustrates the stylet of FIGURE 18A inserted into the proximal section of a catheter;
FIGURE 18D illustrates the stylet of FIGURE 18A inserted into the distal section of the catheter shown in FIGURE 18C;
FIGURE 19A is another embodiment of a stylet suitable for use with the catheter assembly of FIGURES 16A-16C, wherein the stylet includes a preformed distal end region;
FIGURE 19B illustrates the stylet of FIGURE 19A inserted into the distal section of a catheter;
FIGURE 20 is a plan view of the catheter assembly illustrated in FIGURES 16A-16C selectively coupled to a suitable embodiment of an endoscope;
FIGURE 21 is a front elevational view of another exemplary embodiment of a catheter assembly selectively coupled to a suitable embodiment of an endoscope;
FIGURE 22 is a perspective view of another exemplary embodiment of a catheter assembly selectively coupled to a suitable embodiment of an endoscope;
FIGURE 23 is a longitudinal cross-sectional view of one embodiment of a control handle capable of deflecting the distal end of an associated catheter;
FIGURE 24 is a partial view of an alternative embodiment of a steering mechanism suitable for use in the control handle of FIGURE 23;
FIGURE 25 is a plan view of another embodiment of a control handle capable of deflecting the distal end of an associated catheter;
FIGURE 26 is a perspective view of yet another embodiment of a control handle capable of deflecting the distal end of an associated catheter;
FIGURE 27 is a longitudinal cross-sectional view of one embodiment of a control handle, which employs a steering mechanism that multiplies the input movement of an input device into distal tip deflection;
FIGURE 28 is a partial perspective view of another embodiment of a control handle, which employs a steering mechanism that multiplies the input movement of an input device into distal tip deflection;
FIGURES 29-31 illustrate another embodiment of a control handle, which employs a steering mechanism that multiplies the input movement of an input device into distal tip deflection;
FIGURE 32 illustrates one exemplary embodiment of a catheter assembly mounted to an associated endoscope, the catheter assembly being configured so as to reduce potential binding in the catheter when introduced into the endoscope;
FIGURE 33 is a cross-section view of a hinge assembly shown in FIGURE 32;
FIGURE 34 illustrates an embodiment of a catheter assembly of the present invention mounted to an associated endoscope, the catheter assembly being configured so as to reduce potential binding in the catheter when introduced into the endoscope; and
FIGURE 35 illustrates one suitable embodiment of a viewing device.

### DETAILED DESCRIPTION

A plurality of embodiments will now be described with reference to the drawings where like numerals correspond to like elements. The embodiments are directed to systems of the type broadly applicable to numerous medical applications in which it is desirable to insert one or more steerable or non-steerable imaging devices, catheters or similar devices into a body lumen or passageway. Specifically, several embodiments are generally directed to medical devices, systems and components, such as catheters, control handles, steering mechanisms, viewing devices, etc. As mentioned above, the embodiments of the present invention are defined by the features of independent claim 1 and the dependent claims.

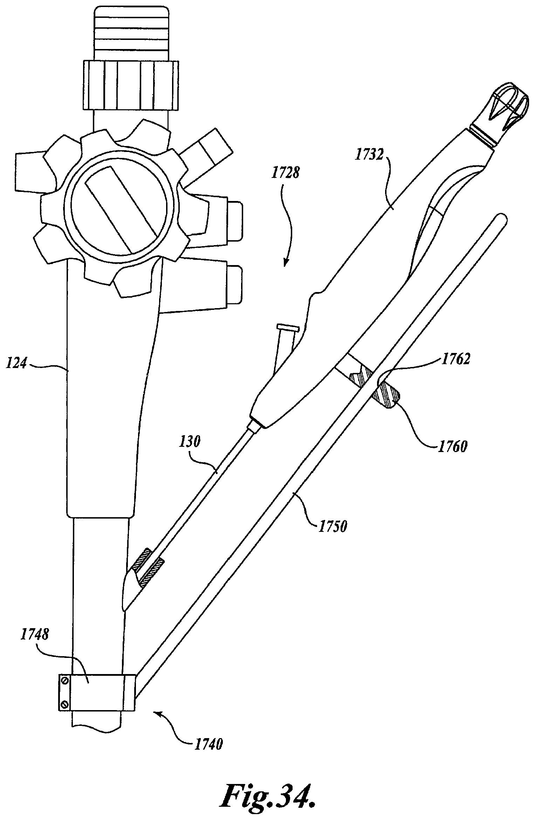

The invention is in particular explained in Fig.34 and the corresponding text in the description.

Several embodiments are generally directed to features and aspects of an *in vivo* visualization system that comprises an endoscope having a
working channel through which a catheter having viewing capabilities is routed. The catheter is preferably of the steerable type so that the distal end of the catheter may be steered from its proximal end as it is advanced into the body. A suitable use for the *in vivo* visualization system includes but is not limited to diagnosis and/or treatment of the duodenum, and particularly the biliary tree. Other embodiments of the present invention are generally directed to features and aspects of the components of an *in vivo* visualization system, including steering mechanisms, control handles, and catheter assemblies, etc.

Several embodiments include medical devices, such as catheters, that incorporate endoscopic features, such as illumination and visualization capabilities, for endoscopically viewing anatomical structures within the body. As such, embodiments can be used for a variety of different diagnostic and interventional procedures. Although exemplary embodiments will be described hereinafter with reference to duodenoscopes, it will be appreciated that the disclosed aspects have wide application, and may be suitable for use with other endoscopes (e.g., ureteroscopes) or medical devices, such as catheters (e.g., guide catheters, electrode catheters, angioplasty catheters, etc.). Accordingly, the following descriptions and illustrations herein should be considered illustrative in nature, and thus, not limiting the scope of the present invention, as claimed. Additionally, the catheter with vision capabilities may be utilized alone, as well as in conjunction with a conventional endoscope.

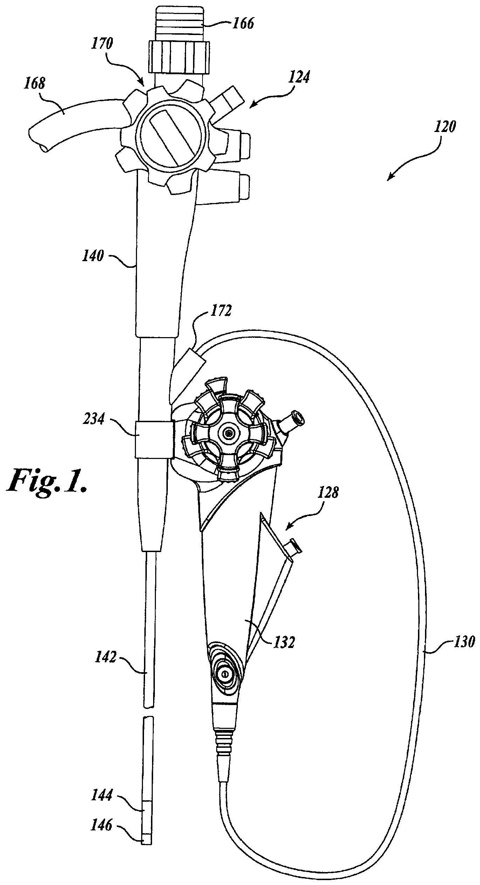

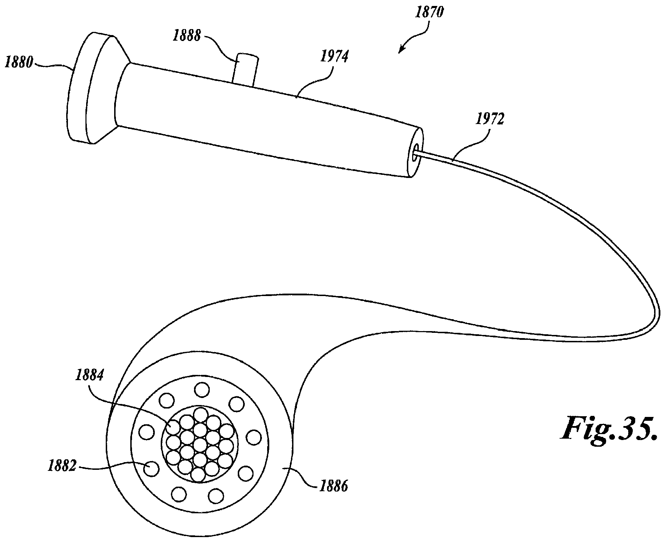

FIGURE 1 illustrates one exemplary embodiment of an *in vivo* visualization system 120. The visualization system 120 includes an endoscope 124, such as a duodenoscope, to which a catheter assembly 128 is operatively connected. As will be described in more detail below, the catheter assembly 128 includes a catheter 130 and a catheter handle 132. The visualization system 120 may further include a viewing device 1870, such as a fiberscope (See FIGURE 35), or other small imaging device that may be routed through a channel of the catheter 130 for viewing objects at the distal end thereof.

In one suitable use, the endoscope 124 is first navigated down the esophagus of a patient and advanced through the stomach and into the duodenum to the approximate location of the entrance to the common bile duct (also known as the papilla). After positioning the endoscope 124 adjacent the common bile duct entrance, the catheter 130
of the catheter assembly 128 is advanced past the distal end of the endoscope 124 and into the common bile duct entrance. Alternatively, the catheter 130 may be routed prior to endoscope insertion. Once inside the common bile duct, the fiberscope allows a physician to view tissue in the bile duct, pancreatic duct and/or intrahepatics for diagnosis and/or treatment.

It will be appreciated that the selection of materials and use of insertable and removable optics in the catheter allow for the catheter to be constructed as a single use device. Once the procedure is performed, the optics can be removed and sterilized for reuse, while the catheter may be removed from the endoscope and discarded.

As best shown in FIGURE 1, one suitable embodiment of an endoscope 124 includes an endoscope handle 140 and an insertion tube 142. The insertion tube 142 is an elongated flexible body that extends from the distal end of the endoscope handle 140. In one embodiment, the insertion tube 142 includes an articulation section 144 disposed at its distal region and a distal tip 146. The insertion tube 142 is constructed of well known materials, such as polyether block amides (e.g., Pebax®), polyurethane, polytetrafluoroethylene (PTFE), and nylon, to name a few.

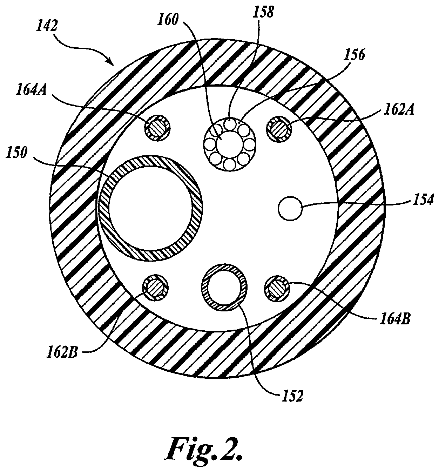

As best shown in the cross-sectional view of FIGURE 2, the insertion tube 142 defines a working channel 150 that extends the entire length thereof and allows for the passage of various treatment or diagnostic devices, such as guide wires, biopsy forceps, and the steerable catheter 130 (FIGURE 1). The insertion tube 142 also includes one or more lumens for the purpose of facilitating the insertion and extraction of fluids, gases, and/or additional medical devices into and out of the body. For example, the insertion tube 142 may include irrigation and/or insufflation lumen 152, and an optional suction lumen 154. The insertion tube 142 further includes one or more lumens for the purpose of providing endoscopic viewing procedures. For example, the insertion tube 142 includes one or more lumens 156 that extend the entire length of the catheter and allows for light and optical fiber bundles 158 and 160 to be routed to the distal end thereof. Alternatively, the insertion tube 142 may include one or more LEDs and an image sensor, such as a CCD or CMOS, for capturing images at the distal tip and transmitting them to the endoscope handle 140. Finally, the insertion tube 142 includes at least one pair of steering wires 162a and 162b, and preferably two pairs of steering wires 162a, 162b and 164a, 164b that are connected at the insertion tube's distal tip and terminate through the proximal end of the insertion tube 142. It will be appreciated that the insertion tube 142 may include other features not shown but well known in the art.

Returning to FIGURE 1, the proximal end of the insertion tube 142 is functionally connected to the distal end of the endoscope handle 140. At the proximal end of the endoscope handle 140, there is provided an ocular 166 through which a user can view the images communicated by the optical fiber bundle 160 (see FIGURE 2) and a light cable 168 (see FIGURE 1) for connecting to an external source of light. While the endoscope shown in FIGURE 1 includes an ocular, the endoscope may be of the electronic type in which the ocular may be omitted and the images obtained from the distal end of the endoscope are transmitted to a video processor via the light cable 168 or other suitable transmission means, and displayed by a suitable display device, such as an LED monitor. Light from the light source can be transmitted to the distal end of the insertion tube 142 via the light fiber bundle 158. The endoscope handle 140 also includes a steering mechanism 170, as shown in the form of control knobs, that are connected to the steering wires 162a, 162b, and 164a, 164b (see FIGURE 2) in a conventional manner for deflecting the distal end of the insertion tube 142 in one or more directions. The endoscope handle 140 further includes a biopsy port 172 connected in communication with the working channel of the insertion tube 142 for providing access to the working channel of the insertion tube 142 from a position exterior the endoscope handle 140.

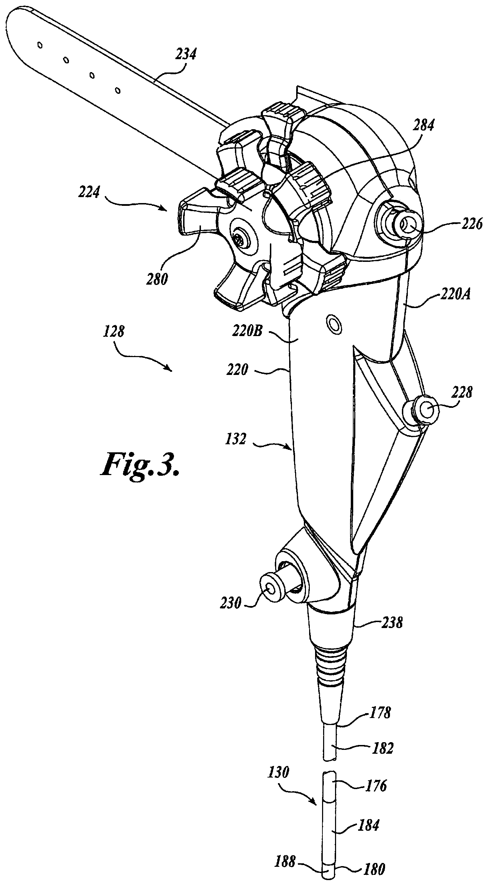

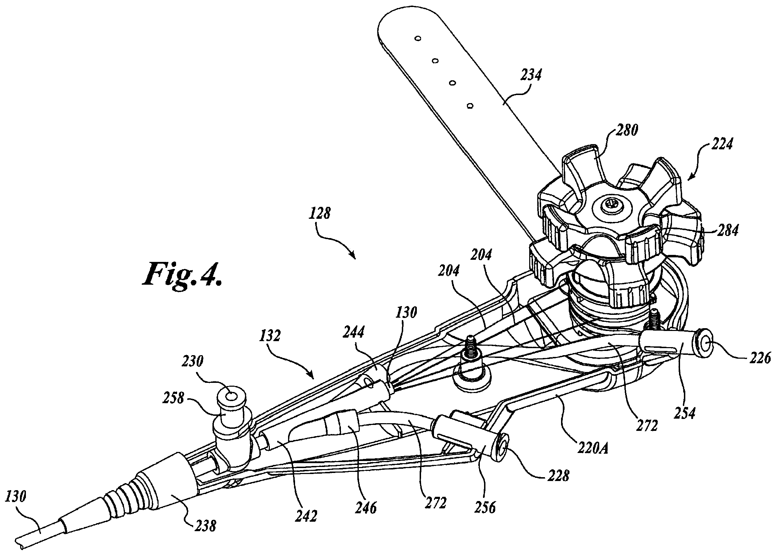

The *in vivo* visualization system 120 further includes the steerable catheter assembly 128 which will now be described in more detail. As best shown in FIGURES 3 and 4, one suitable embodiment of the catheter assembly 128 includes a catheter handle 132 from which the catheter 130 extends. The catheter 130 includes an elongated, preferably cylindrical, catheter body 176 that extends the entire length of the catheter 130 from the catheter proximal end 178 to the catheter distal end 180. In one embodiment, the catheter body 176 has an outer diameter between approximately 5 and 12 French, and preferably between approximately 7 and 10 French. The catheter body 176 may be constructed from any suitable material, such as Pebax® (polyether block amides), nylon, polytetrafluoroethylene (PTFE), polyethylene, polyurethane, fluorinated ethylene propylene (FEP), thermoplastic elastomers and the like, or combinations thereof. The body 176 may be formed of a single material using known techniques in the art, such as extrusion, or multiple materials by joining multiple extruded sections by heat bonding, adhesive bonding, lamination or other known techniques. According to an example, the distal portion of the catheter (approximately 1-2 inches where the flexing occurs) is made more flexible (i.e., less stiff) than the remainder of the catheter.

In the embodiment shown in FIGURE 3, the catheter body 176 includes a proximal section 182 that extends the majority of the catheter 130, a deflection section 184, and a distal tip section 188. The catheter 130 preferably varies in stiffness between the proximal section and the distal tip section. More preferably, the proximal section 182 is stiffer than the deflection section 184. This allows the catheter to be easily advanced without compressing and with minimal twisting while providing deflection capabilities to the deflection section 184 for deflecting the distal end 180. In one embodiment, the proximal section 182 has a durometer value between 35 and 85 shore D, preferable 60-80 shore D, and the deflection section 184 has a durometer value between 5 and 55 shore D, preferable 25-40 shore D.

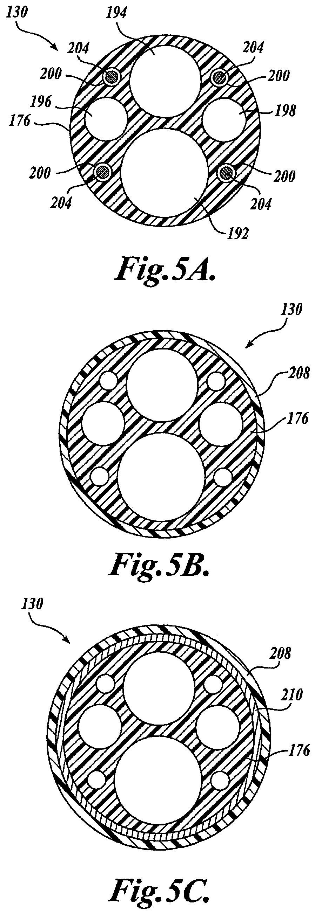

FIGURE 5A is a cross-sectional view of one embodiment of the catheter body 176. The catheter body 176 defines a working channel 192 that extends the length of the catheter and allows for the passage of various treatment or diagnostic devices, such as guide wires, stone retrieval baskets, lasers, biopsy forceps, etc. In one embodiment, the working channel 192 preferably has a diameter sufficient to accept up to a 4-French working device, such as biopsy forceps. The catheter body 176 may also include a channel 194 that extends the entire length of the catheter through which a fiberscope, fiber optic cable, optical assembly or other small diameter viewing device (e.g., 0.25 mm-1.5 mm diameter) can be routed to the distal end of the catheter 130. The catheter body 176 may further include additional channels 196, 198 for use, e.g., as irrigation channels or additional working channels. The channels 196, 198 each extend the entire length of the catheter and, like the working channel 192, allow the passage of devices, liquids and/or gases to and from the treatment area. These channels 196, 198 each have a diameter similar to or smaller than the main working channel, and may be symmetrically positioned to balance the remaining channels during extrusion. Such positioning of the channels balances out the wall thickness and stiffness in two transverse directions. Finally, the catheter body 176 may include one or more steering wire lumens 200 that extend the entire length of the catheter.

Referring to FIGURES 4 and 5A, the catheter 130 further includes one or more steering wires 204 that cause the distal end 180 of the catheter 130 to deflect in one or more directions. The steering wires 204 are routed through a corresponding number of steering wire lumens 200, extend from the distal end 180 of the catheter 130 to the opposing, proximal end 182 of the catheter 130, and terminate in a suitable manner with the steering mechanism, as will be described in detail below. The steering wires 204 may be attached to the distal tip section 188 of the catheter 130 in a conventional manner, such as adhesive bonding, heat bonding, crimping, laser welding, resistance welding, soldering, or other known techniques, at anchor points such that movement of the wires causes the distal end 180 to deflect in a controllable manner. In one embodiment, the steering wires 204 are attached via welding or adhesive bonding to a fluoroscopy marker band (not shown) fixedly attached to the distal tip section. In one embodiment, the band may be held in place via adhesive and/or an outer sleeve, as will be described in more detail below. The steering wires 204 preferably have sufficient tensile strength and modulus of elasticity that they do not deform (elongate) during curved deflection. In one embodiment, the steering wires are made from 304 stainless steel with an 0.008-inch diameter and have a tensile strength of approximately 325 KPSI. The steering wires 204 can be housed in a PTFE thin-walled extrusion (not shown) to aid in lubricity and prevent the catheter 130 from binding up during deflections, if desired.

In the illustrated embodiment shown in FIGURE 5A, the catheter 130 includes two pairs of steering wires 204 that controllably steer the catheter 130 in two perpendicular planes. In alternative embodiments, the catheter 130 includes one pair of steering wires 204 that allow the user to steer the distal tip in one plane. In one embodiment, two steering wires may be provided and are located on opposite sides of the catheter 130 and slide within grooves, as opposed to steering wire lumens 200, formed in the elongated body 176 or either the sheath or outer sleeve, if included, as will be described in more detail below. In a further embodiment, the catheter 130 only includes one steering wire 204 that allows the user to steer the distal tip in one direction. In another embodiment, the steering wires may be omitted, and thus, the catheter 130 can be of a non-steerable type. In such an embodiment, the catheter can be advanced over a guidewire (not shown) pre-placed, e.g., in the bile or pancreatic duct.

In one embodiment, the catheter 130 may also include an outer sleeve 208 that encases the length of the elongated body 176, as shown in cross-section in FIGURE 5B, or sections thereof. The outer sleeve 208 may comprise one of any number of polymer jackets that are laminated, coextruded, heat shrunk, adhesive bonded, or otherwise attached over the catheter body 176. Suitable materials for the sleeve 208 include, but are not limited to, polyethylene, nylon, Pebax® (polyether block amides), polyurethane, polytetrafluoroethylene (PTFE), thermoplastic elastomers, to name a few. The outer sleeve 208 may be used to vary the stiffness of the catheter, if desired, or to provide improved torque transfer and/or other desirable catheter properties. Additionally, the sleeve 208 may be used as one convenient method for securing a more flexible deflection section to the proximal section, as will be described in detail below. In several embodiments, the external surface of the sleeve 208 may have a hydrophilic coating or a silicon coating to ease the passage of the device *in vivo.*

In other embodiments, the catheter 130 may optionally include an inner reinforcement sheath 210 disposed between the elongated body 176 and the outer sleeve 208. The reinforcement sheath encases the length of the elongated body 176 or portions thereof, as shown in FIGURE 5C. The sheath 210 may be a woven or layered structure, such as a braided design of fine wire or polymeric elements (0.001 inches to 0.010 inches in diameter), woven or coiled together along the longitudinal axis of the catheter with conventional catheter braiding techniques. This allows the catheter to be advanced to the desired anatomical site by increasing the column strength of the assembly while also increasing the torsional rigidity of the catheter. Conventional coiled polymer or braid wire may also be used for this component with coil wire dimensioning ranging in width from 0.002 to 0.120 inches and thicknesses from 0.002 to 0.10 inches. Braided ribbon wire may also be used for the sheath. In one embodiment, as will be described in more detail below, the outer sleeve 208 is coextruded, coated, or otherwise attached once the reinforcement layer 210 is applied, to lock the reinforcement layer in place and secure it to the catheter body 176, thereby forming a composite catheter.

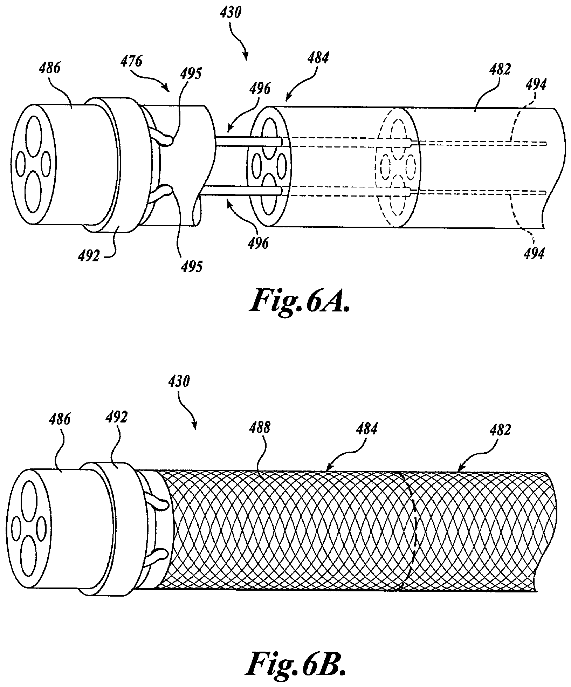

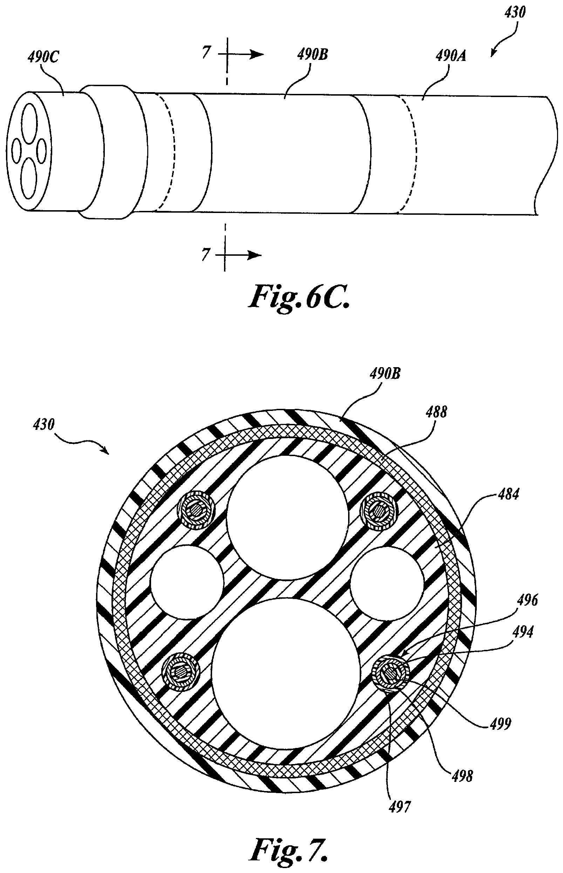

FIGURES 6A-6C, and 7 illustrates one suitable embodiment of a catheter 430 that may be used with the visualization system described above. As best shown in FIGURE 6A, the catheter 430 includes a catheter body 476 having a proximal section 482, a deflecting section 484, and a distal tip section 486. In one embodiment, the proximal section 482 is constructed of a material that is stiffer than the deflecting section 484. The proximal section 482 and the deflecting section 484 may be extrusions constructed from any suitable material, such as polyethylene, nylon, Pebax® (polyether block amides), polyurethane, polytetrafluoroethylene (PTFE), and thermoplastic elastomers, to name a few. In one preferred embodiment, the proximal section is a multi-lumen, PTFE extrusion approximately 200 to 220 cm in length, and the deflecting section 484 is a multi-lumen, Pebax® extrusion approximately 2 to 10 cm in length. The deflection section 484 may be coupled to the proximal section 482 via suitable adhesive or joined by other techniques. The distal tip section 486 may be coupled to the distal end of the deflection section 484 via suitable adhesive. The distal tip section 486 may be constructed of any suitable material, such as stainless steel or engineering plastics, including but not limited to polyethylene, nylon, Pebax® (polyether block amides), polyurethane, polytetrafluoroethylene (PTFE), and thermoplastic elastomers. The catheter body 476 may also include a radio opaque marker band 492 that encircles a portion of the distal tip section 486.

The catheter 430 (see FIGURE 6B) also includes a reinforcement sheath 488 that extends from the proximal end of the catheter to or immediately proximal of the radio opaque marker band 492. The sheath 488 may be a woven or layered structure, such as a braided design of fine wire or polymeric elements (0.001 inches to 0.010 inches in diameter), woven or coiled together along the longitudinal axis of the catheter with conventional catheter braiding techniques. This allows the catheter to be advanced to the desired anatomical site by increasing the column strength of the assembly while also increasing the torsional rigidity of the catheter. The reinforced catheter body shown in FIGURE 6B is then encased by an outer sleeve 490 comprising one or more sleeve sections 490a, 490b, and 490c, having the same or different stiffness values, as best shown in FIGURE 6C, to form the catheter 430.

Returning to FIGURE 6A, the catheter 430 also includes a plurality of steering wires 494 that extend through channels of the catheter body from the proximal end of the catheter past the deflecting section 484. In one embodiment, the steering wires 494 terminate at the radio opaque marker band 492 to which the steering wires 494 are joined by adhesive bonding, laser welding, resistance welding, soldering, or other known techniques. In this embodiment, the catheter body includes openings 495 formed in the outer surface thereof just proximal the radio opaque marker band 492 via any suitable method, such as skiving. These openings 495 communicate with the steering wire channels so that the steering wires 494 may exit the extruded catheter body and connect to the radio opaque marker band 492, as shown.

In some instances where the catheter body is not extruded or otherwise constructed of PTFE or other friction-reducing materials, it may be desirable to encase the steering wires 494 with a laminate structure 496 for allowing the steering wires 494 to move freely within the catheter body, and in particular, the deflecting section 484, and thus, make the mechanics of actuation as smooth as possible. As best shown in FIGURE 7, the laminate structure 496 is formed by outer jacket 497 constructed of a thermoplastic polymer, such as polyurethane, Pebax®, thermoplastic elastomer, etc., which encases an inner reinforcement member 498, such as a metallic braid (e.g., stainless steel braid having, for example, a 0.0015" x 0.006" helically wound). Inside the reinforcement member 498, is a layer 499 of a friction-reducing material, such as PTFE or FEP tubing, over which the aforementioned layers are formed. In embodiments where the proximal section 482 is extruded or otherwise formed with a friction-reducing material, the laminate structure 496 begins at the intersection of the proximal section 482 and the deflecting section 484 and extends to just proximate the radio opaque marker band 492, as best shown in FIGURE 6A.

The multi-lumen catheters described herein may be extruded using known materials, such as PTFE, Nylon, Pebax®, to name a few. The catheters may be extruded using mandrels. In several examples, the mandrels may be constructed from suitable materials, such as stainless steel, stainless steel with PTFE coating, or a phenol plastic, such as Cellcore®. In the embodiment shown in FIGURE 5A, the multi-lumen catheter 130 has eight lumens that include a working channel 192, a fiberscope or viewing device channel 194, and four smaller steering wire lumens 200 spaced 90 degrees apart. To balance out the wall thicknesses and stiffnesses in the traverse directions during extrusion, left and right lumens 196, 198 may also be formed using separate mandrels. These lumens 196, 198 may be used for air/gas irrigation and insufflation.

The catheter 130 shown in FIGURE 5B may optionally include an outer sleeve 208. The sleeve may be constructed of suitable materials by coextrusion, heat shrinking processes, such as reflow, or spray coating. The outer sleeve 208 may provide additional rigidity, improved torque transfer, etc. In one embodiment, the outer sleeve may be applied for facilitating the attachment of a flexible distal section, such as a deflection section, that has a lower durometer value than the remaining catheter body. In such an embodiment, one suitable material that may be used includes, but is not limited to, Pebax® (polyether block amide). In other embodiments, the catheter 130 may include a reinforcement layer 210 or sheath between the catheter body 176 and the outer sleeve 208, as best shown in FIGURE 5C. The reinforcement may be any known catheter reinforcement structure, such as wire coil or braid. In such as embodiment, the outer sleeve 208 is coextruded, coated, or otherwise attached once the reinforcement layer 210 is applied, to lock the reinforcement layer in place. It will be appreciated that the reinforcement layer 210 may extend the entire length of the catheter or portions thereof. In one embodiment, the reinforcement layer 210 extends over the deflection section. It will be appreciated that if the body is extruded from PTFE, its outer surface should be etched or otherwise prepared for appropriate bonding with the outer layer.

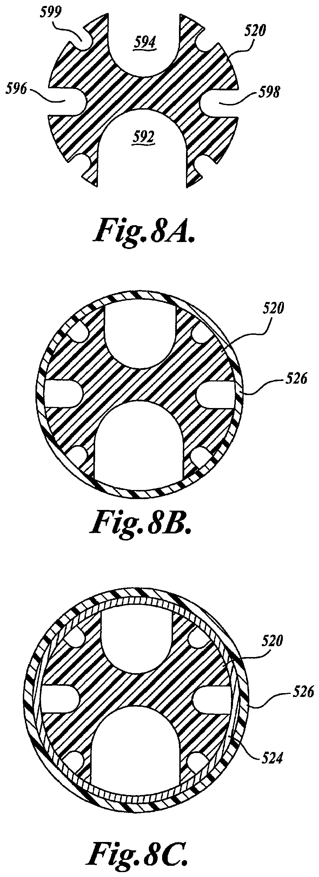

In accordance with another embodiment, the catheter may be built up using a catheter core 520, an optional reinforcement layer 524, and an outer sheath or jacket 526, as best shown in FIGURES 8A-8C. The catheter core 520 is an open-lumen core that is extruded from suitable materials, such as nylon, PTFE, Pebax®, etc., with the use of mandrels. In this embodiment, the mandrels (not shown) are placed and configured to produce a plurality of open-lumens 592, 594, 596, 598, and 599 when extruded. The mandrels may be constructed from metal, Cellcore®, or PTFE. Once the open-lumen core has been extruded, the mandrels are kept in place and the core is either coextruded to add the outer sleeve 526, as shown in FIGURE 8B, or braided and coextruded to add a reinforcement layer 524 and an outer sleeve 526, as shown best in FIGURE 8C. As was discussed above, the outer sleeve 526 may function to lock the braid in place and/or to facilitate attachment of a distal section, such as a deflection section, having, for example, a lower stiffness value, if desired.

The mandrels (not shown) can then be removed after coextrusion. In one embodiment, the mandrels are constructed of a phenol plastic, such as Cellcore®. To remove these mandrels, the mandrels are pulled from one or both ends. Due to the "necking down" effect inherent to the Cellcore® material, the cross-sectional areas of the mandrels decrease when pulled in tension, thereby allowing the mandrels to be removed from the built-up catheter. In one embodiment, this property of Cellcore® may be used to the manufacture's advantage by using such a material for the steering wire lumen mandrels. However, instead of completely removing the mandrels from the steering wire lumens, tension forces may be applied to the steering wire mandrels, and the mandrels may be drawn to a decreased diameter that will be sufficient to function as the steering wires. Thus, to be used as steering wires, the drawn mandrels are then connected to the distal end of the catheter in a conventional manner. While the latter embodiment was described as being coextruded to form the outer sheath, the outer sheath may be formed on the catheter core by a heat shrink process or spray coating.

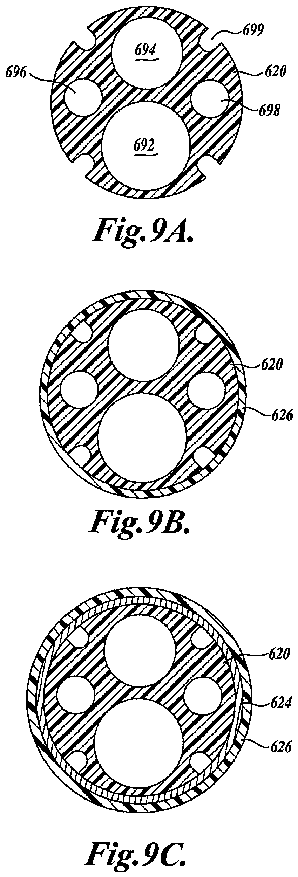

It will be appreciated that not all of the lumens in the latter embodiments need to be formed as open-lumens. Thus, as best shown in FIGURES 9A-9C, only the steering wire lumens 699 are formed as open-lumens. This will create oversized lumens for the steering wires and provided the largest possible lumen diameters for the lumens 692, 694, 696, and 698.

As was described above, in several embodiments of the catheter, it is desirable for the deflection section to be configured to deflect more easily than the proximal section. In one embodiment, the deflection section has a durometer value less than the proximal section. In other embodiments, the flexibility may be varied gradually (e.g., increasingly) throughout the length of a catheter tube from its proximal end to its distal end. In other embodiments, the deflection section may be an articulating joint. For example, the deflection section may include a plurality of segments that allow the distal section to deflect in one or more directions. For examples of articulation joints please see co-pending U.S. Patent Application Nos. 10/406,149, 10/811,781, and 10/956,007.

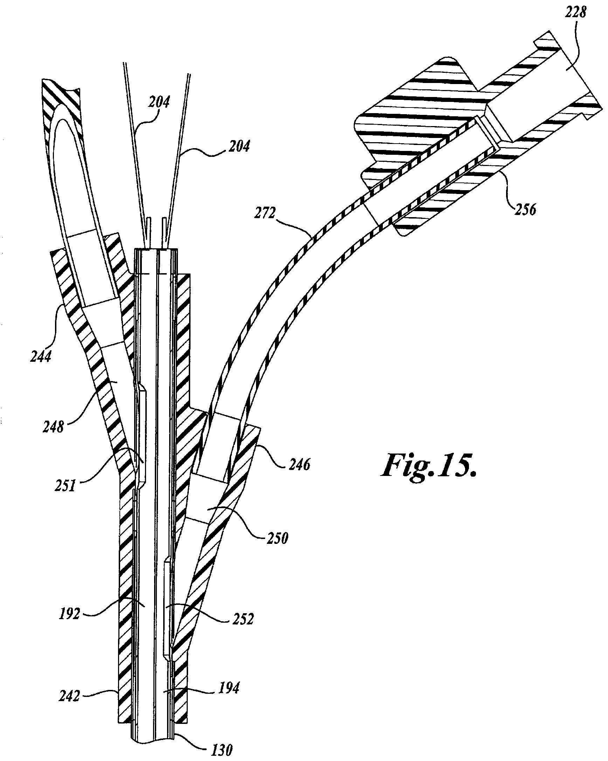

Returning to FIGURES 3 and 4, the catheter 130 is functionally connected to the catheter handle 132. The handle 132 includes a handle housing 220 to which a steering mechanism 224, one or more ports 226, 228, 230, and an endoscope attachment device 234 is operatively connected. In one embodiment, the handle housing 220 is formed by two housing halves 220a and 220b joined by appropriate removable fasteners, such as screws, or non-removable fasteners, such as riveting, snaps, heat bonding, or adhesive bonding. In the embodiment shown, the proximal end of the catheter 130 is routed through a strain relief fitting 238 secured at the distal end of the handle housing 220 and terminates at a Y connector 242, as best shown in FIGURES 4 and 15. The Y connector 242 may be secured to the handle housing 220 via any suitable means, such as adhesive bonding. Similarly, the proximal end of the catheter 130 is securely coupled to the Y connector 242 via suitable means known in the art, such as adhesive bonding. The Y connector 242 includes first and second branch fittings 244 and 246 that define respective passageways 248 and 250 for communicating with the catheter working channel and the catheter imaging device channel, respectively, through openings 251 and 252 located on the outer surface of the catheter, as best shown in FIGURE 15.

In some examples, the openings 251 and 252 may be formed by skiving the outer surface of the catheter. This process may be done manually using known mechanical techniques, or may be accomplished by laser micro-machining that removes a localized area of material from the outer surface of the catheter to expose one or more catheter channels. When assembled, the proximal ends of the catheter channels are plugged by adhesive, or the proximal end of the catheter is capped to prohibit access to the channels.

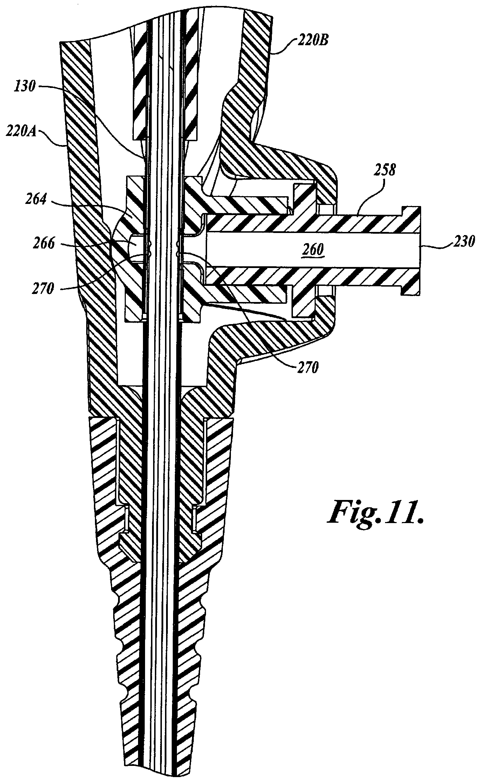

As was described above, the handle housing 220 includes one or more ports 226, 228, 230 for providing access to the respective channels of the catheter 130. In the embodiment shown, the ports include, but are not limited to, a working channel port 226, an imaging device port 228, and an irrigation/suction port 230. The ports may be defined by any suitable structure. For example, the working channel port 226 and the imaging device port 228 may be defined by fittings 254 and 256, respectively, that may be bonded or otherwise secured to the handle housing 220 when assembled. In one embodiment, the housing halves may define cooperating structure that securely locks the fittings 254 and 256 in place when assembled. With regard to the irrigation/suction port 230, a luer style fitting 258 is preferably used for defining the port 230. The fitting 258 defines a passageway 260 for fluidly connecting the port 230 with the appropriate catheter channels, as best shown in FIGURE 11. The fitting 258 works in conjunction with a barrel connector 264 that ensconces the catheter 130. The barrel connector 264 defines a cavity 266 that surrounds the perimeter of the catheter 130 and is fluidly connected to the appropriate catheter channels (irrigation channels) via inlets 270. As such, the port 230 is connected in fluid communication with the irrigation channel via passageway 260 and cavity 266. In one embodiment, the inlets 270 are formed by skiving the outer surface of the catheter. This process may be done manually using known mechanical techniques, or may be accomplished by laser micro-machining that removes a localized area of material from the outer surface of the catheter to expose one or more catheter channels. The working channel port 226 and the imaging device port 228 are connected in communication with the branch fittings 254 and 256 of the Y connector, respectively, via appropriate tubing 272, and best shown in FIGURE 4.

The catheter handle 132 also includes a steering mechanism 224. The steering mechanism 224 of the catheter handle 132 controls deflection of the distal end 180 of the catheter 130. The steering mechanism 224 may be any known or future developed mechanism that is capable of deflecting the distal end of the catheter by selectively pulling the steering wires. In the embodiment shown in FIGURES 3 and 4, the steering mechanism 224 includes two rotatable knobs for effecting 4-way steering of the catheter distal end in the up/down direction and in the right/left direction. This mechanism 224 includes an outer knob 280 to control up/down steering and an inner knob 284 to control right/left steering. Alternatively, the inner knob 284 may function to control right/left steering and an outer knob 280 may function to control up/down steering. The knobs are connected to the distal end of the catheter 130 via the steering wires 204, respectively, that extend through the catheter 130. While a manually actuated steering mechanism for effecting 4-way steering of the distal is shown, it will be appreciated that a manually actuated steering mechanism that effects 2-way steering may be practiced with.

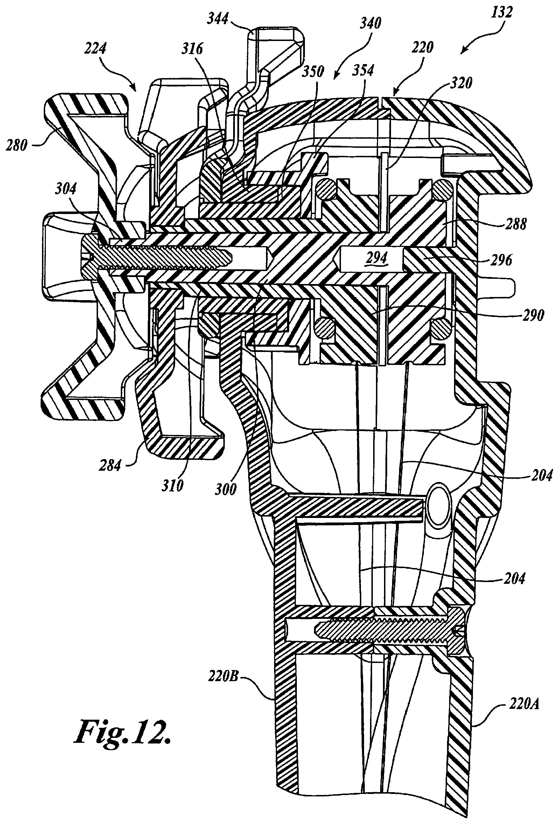

Referring now to FIGURE 12, there is shown one embodiment of the steering mechanism 224. The steering mechanism 224 includes inner and outer pulleys 288 and 290, and control knobs 280 and 284. The inner pulley 288 for left and right bending control is mounted via an inner bore 294 for rotation on a shaft 296 integrally formed or otherwise positioned to extend into the interior of the handle housing 220 in a fixed manner from the housing half 220a. The inner pulley 288 is integrally formed or keyed for rotation with one end of an inner rotary shaft 300. The opposite end of the inner rotary shaft 300 extends outside the handle housing 220 to which the control knob 280 is attached for co-rotation. In one embodiment, the end 304 of the inner rotary shaft 300 is configured to be keyed with a cooperatingly configured control knob opening. The control knob 280 may then be retained thereon via a threaded fastener. The proximal end of one pair of steering wires 204 are connected to opposite sides of the inner pulley 288 in a conventional manner.

The outer pulley 290, for up and down bending control, is rotatably fitted over the inner rotary shaft 300 for independent rotation with respect to the inner pulley 288. The outer pulley 290 is integrally formed or keyed for rotation with one end of an outer rotary shaft 310. The outer rotary shaft 310 is concentrically arranged in a rotational manner over the inner rotary shaft 300. The opposite end of the outer rotary shaft 310 extends outside the handle housing 220 to which the control knob 284 is attached for co-rotation. The rotary shafts 300, 310 are further supported for rotation within the housing 220 by a boss 316 integrally formed or otherwise positioned to extend inwardly into the handle housing 220 from the housing half 220b. It will be appreciated that other structure may be provided that rotatably supports the pulleys 288, 290 and shafts 300, 310 within the handle housing 220. When assembled, the proximal ends of the second pair of steering wires 204 are fixedly connected in a conventional manner to the outer pulley 290, respectively.

In one embodiment, a thrust plate 320 is positioned between the inner and outer pulleys 288, 290 for isolating rotary motion therebetween. The thrust plate 320 is restricted from rotation when assembled within the housing 220.

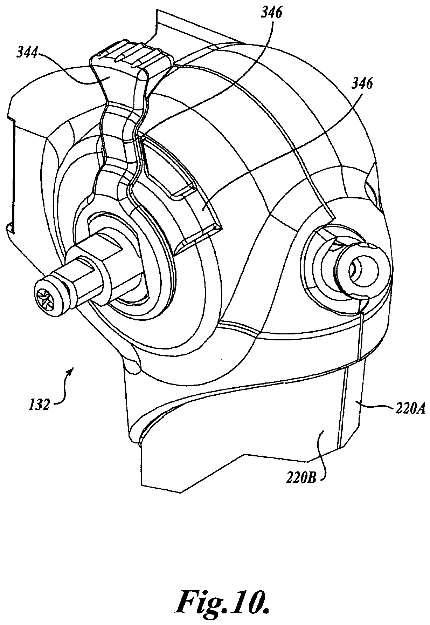

The steering mechanism 224 may further include a lock mechanism 340 that functions to lock the catheter 130 in a desired deflection position during use. The lock mechanism 340 includes a lever 344 that is actuatable between a locked position and an unlocked position. In the embodiment shown in FIGURE 10, detents 346 are provided, and may be molded into the exterior housing half 220b to index the movement between the locked and unlocked positions. A small protuberance (not shown) may be included to signal the user that the lever 344 has changed positions.

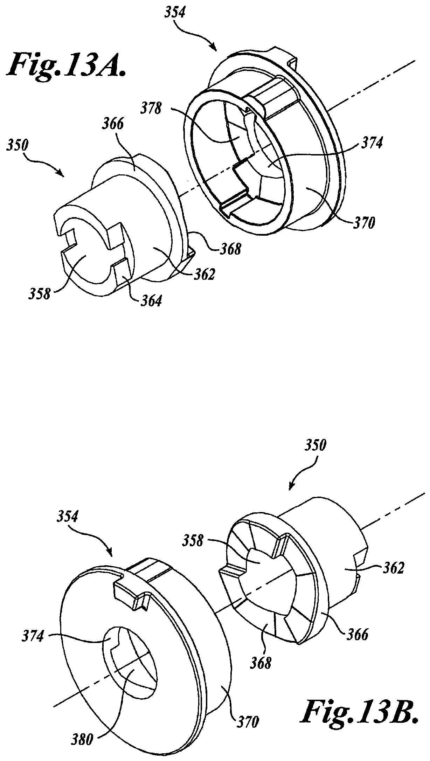

Referring now to FIGURES 12, 13A, and 13B, the lock mechanism 340 further includes a lever member 350 and a pulley member 354 that are housed within the handle housing 220 when assembled. The lever member 350 includes a throughbore 358 that is sized and configured for receiving the outer rotary shaft 310 in a rotationally supporting manner. The lever member 350 includes a boss section 362 that is sized and configured to be rotationally supported by the inwardly extending boss 316 when assembled. The boss section 362 is configured at one end 364 to be keyed for rotation with one end of the lock lever 344. The lever member 350 further includes a flange 366 integrally formed at the other side of the boss section 362. The end face 368 of the flange 366 defines a cam profile that annularly extends around the perimeter of the flange 366. In the embodiment shown, the cam profile is formed by varying the thickness of the flange. The pulley member 354 includes a boss section 370 that is sized and configured for receiving the lever member 350 therein. The pulley member 354 includes an inwardly extending flange 374 that defines a cam profile on the lever member facing surface 378 of the flange 374. Similar to the lever member 350, the cam profile of the pulley member 354 is formed by varying the thickness of the flanges as it annularly extends. The inwardly extending flange 374 further defines a throughbore 380 that is sized and configured for receiving the outer rotary shaft 310 in a rotationally supporting manner. When assembled, the pulley member 354 is restricted from rotating with respect to the housing 220 but allowed to linearly translate, as will be described in more detail below.

When assembled, the lever member 350 is inserted within the pulley member 354, the cam profiles mate, and the lever 344 is keyed for rotation to the lever member 350. The cam profiles on the lever member 350 and the pulley member 354 are specifically configured to transmit a rotary motion of the lever 344 into translational movement of the pulley member 354. Thus, when the lever member 350 rotates by movement of the lever 344 from the unlocked position to the locked position, the pulley member 354 moves away from the lever member 350 in a linear manner by coaction of the cam profiles. Therefore, the lever member 350 acts like a cam, and the pulley member 354 acts like a follower to convert rotary motion of the lever 344 into linear motion of the pulley member. The linear movement of the pulley member 354 causes the inner pulley 288 to frictionally engage the housing 220 and the thrust plate 320, while the outer pulley 290 frictionally engages the thrust plate on one side and the pulley member of the other. The friction present between the engaged surfaces prohibits rotation of the inner and outer pulleys 288 and 290, and thus, locks the distal end of the catheter in a deflected position.

To change the deflection of the distal end of the catheter from one position to another, the lock lever 344 is moved from the locked position to the unlocked position. This, in turn, rotates the lever member 350 with respect to the pulley member 354. Due to the configuration of the cam profiles of the lever and pulley members, the pulley member 354 is capable of moving toward the lever member 350. This alleviates the friction between the engagement surfaces and allows the inner and outer pulleys 288 and 290 to rotate by turning the control knobs 284 and 280.

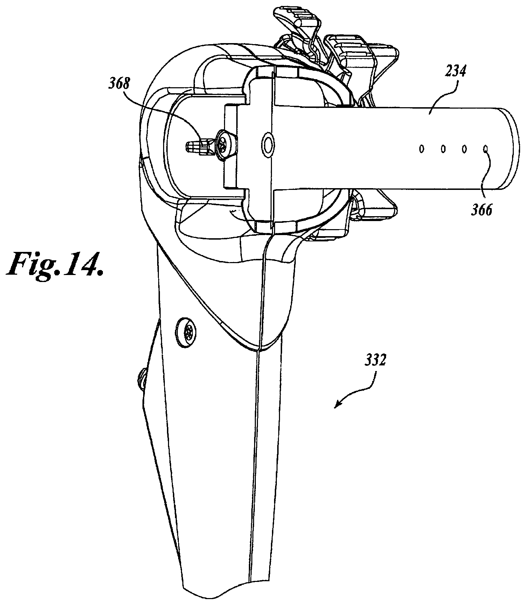

The catheter assembly 128 can be mounted directly to the endoscope handle 140 so that a single user can manipulate both the endoscope 124 and the catheter assembly 128 using two hands. In the embodiment shown, the catheter handle 132 is attached to the endoscope 124 via the endoscope attachment device, such as the strap 234. The strap 234 can be wrapped around the endoscope handle 140, as best shown in FIGURE 1. The strap 234 includes a number of notches 366 into which the head of a housing projection 368 is selectively inserted to couple the catheter handle to the endoscope, as best shown in FIGURE 14. The strap 234 allows the catheter handle 132 to rotate around the shaft of the endoscope 124, if desired. The strap 234 is positioned such that when used to attach the handle 132 to the endoscope 124, the longitudinal axes of both handles are substantially aligned, as shown best in FIGURE 1. Additionally, the strap orientation and the location of the ports on the catheter handle 132 allow for manipulation of diagnostic or treatment devices and viewing devices through the catheter without interfering with control and use of the endoscope. As a result of directly connecting the catheter assembly 128 to the endoscope 124, as shown in FIGURE 1, the catheter 130 creates a loop, known as a service loop, prior to entrance into the biopsy port 172. In one embodiment, the catheter may include a proximally located stop sleeve or collar (not shown), which limits the minimum diameter of the service loop and the extension of the catheter 130 beyond the distal end of the conventional endoscope. Alternatively, a mark or indicia may be placed on the catheter 130 and used to prevent overinsertion of the catheter 130.

In embodiments and examples that form a service loop by directly connecting the catheter handle 132 to the endoscope 124, the catheter 130 is preferably constructed to be suitably longer than conventional catheters to compensate for the service loop. In several of these embodiments, the catheter handle 132 is preferably mounted below the biopsy port 172 of the endoscope 124 and the catheter 130 is preferably looped upward and into the biopsy port 172. In this configuration, the catheter 130 is accessible and can be gripped by the user just above the biopsy port for catheter insertion, withdrawal, and/or rotation.

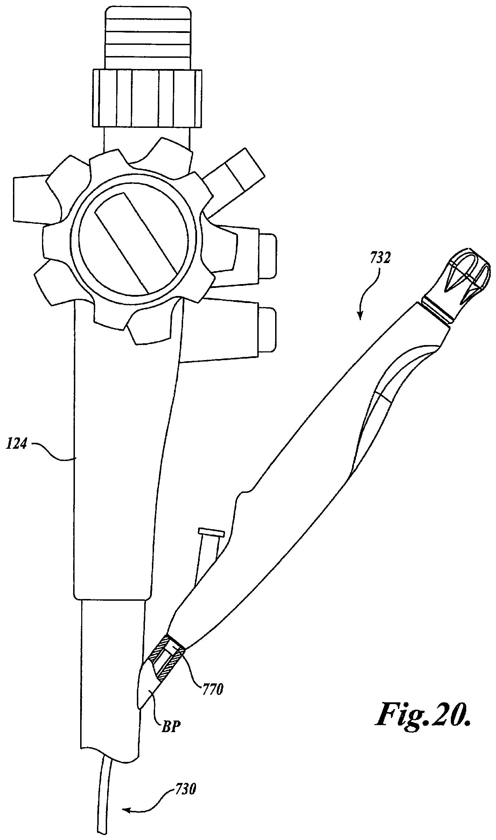

While the embodiment above illustrates a handle connected below the biopsy port and longitudinally oriented with respect to the catheter, other configurations are possible. For example, the handle can be associated with the endoscope so that the longitudinal axis of the catheter handle is substantially transverse to the longitudinal axis of the endoscope handle. Additionally, the catheter handle may be mounted proximally or distally on the biopsy port or may be mounted directly on the biopsy port so that the longitudinal axis of the catheter is coaxial with the biopsy port. Examples of these alternative configurations are described in more detail below with respect to FIGURES 20-22, and 31-34.

As was discussed briefly above, a small diameter viewing device, such as a fiberscope or other imaging device, may be slidably routed through one channel (e.g., imaging device channel) of the catheter 130 (FIGURE 3) to the distal end thereof. The viewing device permits the user of the catheter assembly to view objects at or near the distal end or tip of the catheter. For a detailed description of one suitable embodiment of a viewing device that may be utilized by the visualization system, please see the viewing device described in FIGURE 35 below. For other examples of viewing devices, please see the description of the fiber optic cable in co-pending U.S. Application No. 10/914,411, and the guidewire scope described in U.S. Published Patent Application No. 2004/0034311 A1.

Turning now to FIGURE 35, there is shown one suitable embodiment of a viewing device 1870, such as a fiberscope or other imaging device. As was discussed briefly above, the viewing device 1870 may be slidably routed through one channel (e.g., viewing device or imaging device channel) of the catheter 130 to the distal end thereof. The viewing device 1870 permits the user of the catheter assembly to view objects at or near the distal end or tip of the catheter.

The viewing device 1870 includes a fiber optic cable 1972 connected to an optical handle 1974. The fiber optic cable 1972 is defined, for example, by one or more optical fibers or bundles 1882 and 1884 encased by a cylindrical, elongated tubular sleeve 1886. The outer diameter of the fiber optic cable 1972 is preferably between 0.4 mm and 1.2 mm, although other sizes may be used, depending on its application and the channel size of the catheter. The tubular sleeve 1886 of the fiber optic cable 1972 may be constructed of any suitable material, such as nylon, polyurethane, polyether block amides, just to name a few. Additionally, a metallic hypotube may be used.

In the illustrated embodiment, the fiber optic cable 1972 includes one or more centrally extending coherent imaging fibers or fiber bundles 1884 and one or more circumferentially extending illumination fibers or fiber bundles 1882 (which may not be coherent) that generally surround the one or more imaging fibers of fiber bundles 1884. The fibers or fiber bundles 1882 and 1884 may be attached to the tubular sleeve 1886 via suitable adhesive. The distal end of the fiber optic cable 1972 includes a distal lens and/or window (not shown) that encloses the distal end to protect the fiber bundles

The proximal end of the fiber optic cable 1972 is functionally connected to the handle 1974. In use, the illumination fibers or fiber bundles 1882 illuminate the area or objects to be viewed, while the imaging fibers or fiber bundles 1884 communicate the illuminated image to an image viewing device, such as an eyepiece or ocular lens device 1880, through which a user can view the images communicated via the imaging fibers or fiber bundles 1884. The optical handle 1974 can also be configured to connect to a camera or imaging system such that users can save images and view them on display. It will be appreciated that the handle 1974 may include other known components, such as adjustment knobs (not shown) that adjust the relative positioning of the lenses and, thus, adjusts the focus of the image transmitted through them. The handle 1974 further includes a light post 1888 that is connected to the proximal end of the illumination fibers or fiber bundle 1882. The light post 1888 is configured to be releasably connected to a light cable for supplying light from a light source external the viewing device 1870 to the illumination fibers or fiber bundle 1882.

The viewing device 1870 may have a stop collar or sleeve (not shown) to limit movement of the cable 1972 through the viewing device channel of the catheter and limit the length by which the cable 1972 can extend beyond the distal tip of the catheter 130. The inner surface of the viewing channel of the catheter may have color markings or other calibration means to indicate to the user when inserting the cable 1972 that the end of the catheter is approaching or has been reached.

One suitable method of operation of the *in vivo* visualization system 120 will now be described in detail with reference to the aforementioned figures. The insertion tube 142 of the endoscope 124 is first navigated down the esophagus of a patient under endoscope visualization. The insertion tube 142 of the endoscope 124 is advanced through the stomach and into the duodenum at the bottom of the stomach. The biliary tree comprises the cystic duct from the gall bladder, the hepatic duct from the liver and the pancreatic duct from the pancreas. Each of these ducts joins into the common bile duct. The common bile duct intersects with the duodenum a slight distance below the stomach. The papilla controls the size of the opening at the intersection between the bile duct and duodenum.

The papilla must be crossed in order to reach the common bile duct to perform a biliary procedure. The insertion tube 142 of the endoscope 124 is navigated under direct visualization so that the exit port of the working channel 150 is directly across from the papilla or so that the port is slightly below the papilla. After positioning the distal end of the insertion tube 142 in the proper position, the catheter 130 with the viewing device 1870 is advanced through the working channel 150 in the endoscope 124 such that the distal end of the catheter 130 emerges from the endoscope and cannulates the papilla. The endoscope 124 provides viewing of the catheter 130 as it emerges from the endoscope 124 and is advanced to enter the papilla. After cannulating the papilla, the catheter 130 may be advanced into the common bile duct. Once advanced into the common bile duct, the fiber optic cable 1972 of the viewing device 1870 located within the catheter 130 allows a physician to view tissue in the bile duct for diagnosis and/or treatment

Alternatively, once the insertion tube 142 of the endoscope 124 is in place next to the papilla, a conventional guidewire and sphinctertome may be advanced together through the endoscope and through the papilla to enter the common bile duct and pancreatic duct. It may be necessary for the physician to use the sphinctertome to enlarge the papilla. The sphinctertome may then be removed from the patient while leaving the conventional guidewire in place. The catheter 130 and the fiber optic cable 1972 of the viewing device 1870 may then be advanced together over the conventional guidewire through the papilla and into the common bile duct. Once inside the common bile duct, the fiber optic cable 1972 of the viewing device 1870 allows a physician to view tissue in the bile duct for diagnosis and/or treatment.

It will be appreciated that the selection of materials and use of insertable and removable optics in the catheter allow for the catheter to be constructed as a single use device. Once the procedure is performed, the optics can be removed and sterilized for reuse, while the catheter may be removed from the endoscope and discarded.

While the steerable catheter assembly 128 has been described above for use with an endoscope, it will be appreciated that the catheter assembly may be used with other devices, or may be used as a stand-alone device or in conjunction with the viewing device 1870.

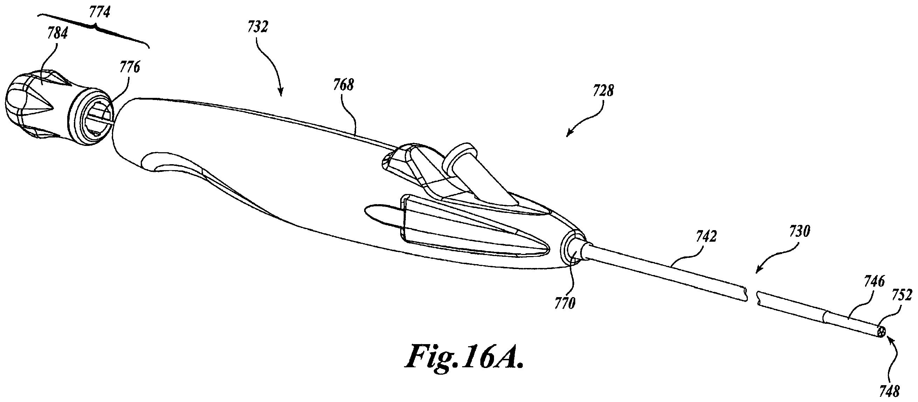

Turning now to FIGURES 16A-16B, there are shown front and rear perspective views, respectively, of an alternative embodiment of a catheter assembly 728. The catheter assembly 728 may be either utilized by the visualization system 120 shown in FIGURE 1 in place of the assembly 128, or may be used independently of the system 120 of FIGURE 1, or may be used with other medical devices, as desired. In this embodiment, the catheter assembly 728 includes an elongated catheter 730 and a control handle 732.

As best shown in FIGURE 16A, the catheter 730 includes a proximal section 742 that extends along the majority of the catheter 730, and a shorter distal section 746. The proximal section 742 has a higher durometer value or stiffness than the distal section 746 so that the distal section 746 is easier to deflect than the proximal section 742. Alternatively, instead of, or in conjunction with, a lower durometer distal section, the catheter 730 may include an articulating section or joint coupled to the end of the proximal section for providing easier deflecting as compared to the proximal section. Several examples of articulation sections or joints that may be practiced with embodiments of the catheter 730 were described above with respect to catheter 130. The catheter 730 may be partially or wholly constructed from any suitable material, such as Pebax® (polyether block amides), nylon, polytetrafluoroethylene (PTFE), polyethylene, polyurethane, fluorinated ethylene propylene (FEP), thermoplastic elastomers and the like, or combinations thereof. In some embodiments, the catheter 730 may be constructed in accordance with one or more aspects of the catheter 130 described above with respect to FIGURES 3 and 5A-5C. In one embodiment, the catheter 730 has an outer diameter between approximately 5 and 12 French.

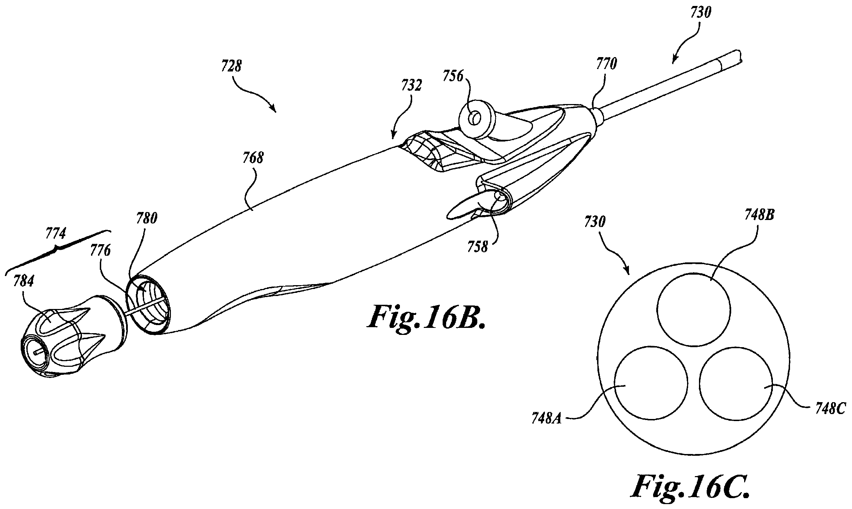

The catheter 730 defines one or more channels 748 extending from its proximal end (not shown) to its distal end 752. In the end view of the catheter 730 shown in FIGURE 16C, the one or more channels 748 of the catheter 730 includes a steering device channel 748a and, for example, a working channel 748b and a fiberscope or other viewing device channel 748c. As shown, the steering device channel 748a is slightly offset from the longitudinal axis of the catheter, although other configurations are possible. It will be appreciated that the catheter 730 may be constructed with other channels (not shown), such as an irrigation/insufflation channel. Further, it will be appreciated that the catheter 730 may omit one or more of the aforementioned channels, as desired.

Referring again to FIGURES 16A-16B, the control handle 732 is functionally connected at its distal end to the proximal end of the catheter 730. The control handle 732 includes a handle housing 768 with one or more ports, such as a working channel port 756 and a viewing device port 758 (See FIGURE 16B). The ports 756 and 758 are configured for accessing one or more of the plurality of channels 748 defined by the catheter 730, such as the working channel 748b and the viewing device channel 748c, respectively. The control handle 732 may include a manifold and associated structure, such as flexible conduits, to interconnect the ports and the catheter channels. For example, in one embodiment, the manifolds, flexible conduits, etc., are arranged and configured substantially similar to the Y connector described above with respect to FIGURE 15.

The control handle 732 further includes a steering mechanism 774 for controlling the deflection of the distal end 752 of the catheter 730. In the embodiment shown in FIGURES 16A-16C, the steering mechanism 774 comprises an elongated wire or stylet 776 and a knob 784. An opening 780 is provided at the proximal end of the handle housing 768. The opening 780 is connected in communication with the steering device channel 748a of the catheter 730 via a passageway defined by, for example, the handle housing 768. The opening 780, the passageway, and the steering device channel 748a are configured for allowing passage of the stylet 776 through the control handle 732 and the catheter 730 in a slidable and axially rotational manner. The knob 784 is attached to the proximal end of the stylet 776, and may be utilized for controlling movement of the stylet with respect to the handle housing 768, as will be described in more detail below.

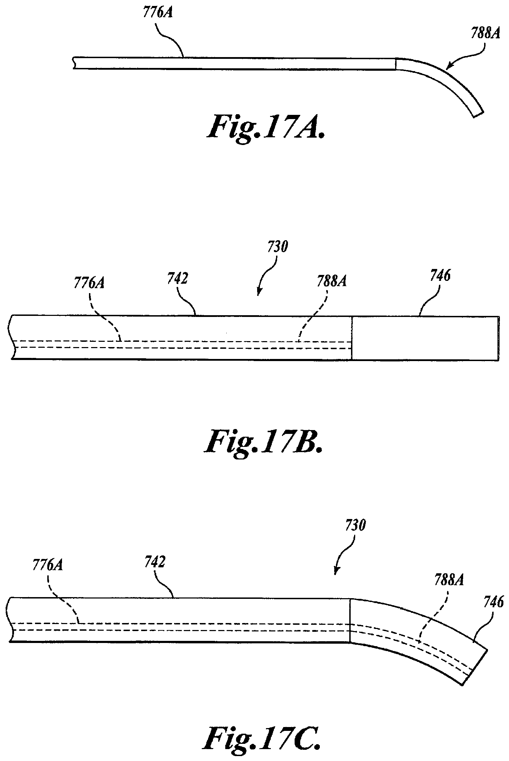

The stylet 776 may have many configurations for controlling the deflection of the distal end 752 of the catheter 730. FIGURE 17A illustrates a partial view of one exemplary embodiment of a stylet 776a. In this embodiment, the stylet 776a is a preformed shaped wire constructed of a material that exhibits either non-linear or linear superelastic properties. One example of a material that exhibits either non-linear or linear superelastic properties is a nickel titanium alloy, such as Nitinol®. In the embodiment shown, the preformed shape of the stylet 776a includes a bent distal end region 788a, as shown best in FIGURE 17A. Although the bent distal end region 788a has been shown as slightly arcuate, it will be appreciated that the bent distal end region 788a of the stylet 776a may be formed in any arcuate and/or linear orientation, depending on the procedure to be conducted. The stylet 776a may be provided with the control handle 732 or may be sold separately as one in a set of preformed stylets, each having a different preformed profile.

The bent distal end region 788a of the stylet 776a may be created in one embodiment by restraining the stylet 776a in the desired shape and heating the wire to approximately 500°C for a suitable period of time, e.g., 10 minutes. The stylet 776a is then allowed to cool. Upon cooling, the stylet 776a retains the preformed distal end region shape. Stress may then be applied to the stylet 776a to change its shape. For example, a straightening force may be applied to the preformed distal end region 788a of the stylet 776a to permit introduction of the stylet 776a into the proximal end of the catheter 132. Such stress applied to the stylet 776a generates internal forces, hereinafter referred to as restoring forces, in the stylet. Because of the superelasticity of the stylet 776a, once the straightening forces are removed, as will be described in detail below, the restoring forces generated with the stylet 776a return the stylet 776a to its original preformed shape.

It will be appreciated that the materials, configurations, and dimensioning of both the catheter 730 and the stylet 776a may be selected such that the proximal section 742 of the catheter 730 provides an appropriate restraining force (e.g., straightening force) against the straightened stylet for keeping the stylet 776a from returning to its preformed shape when positioned therein, as best shown in FIGURE 17B. Further, it will be appreciated that the materials, configurations, and dimensioning of the catheter may be selected such that the restoring force (induced when the stylet was straightened and maintained while positioned in the proximal section) of the stylet 776a overcomes the stiffness of the distal section 746 of the catheter 730 when positioned therein and deflects the catheter 730 according to its preformed shape, as best shown in FIGURE 17C.

When assembled, deflection of the distal end 752 of the catheter 730 may be controlled by the knob 784. By advancing the knob 784 toward the control handle 732, the preformed stylet 776 is advanced from the stiffer proximal section 742 of the catheter 730 shown in FIGURE 17A to the more flexible distal section 746 shown in FIGURE 17C. This advancement deflects the distal end 752 of the catheter 730 in accordance with the preformed bent distal end region 788a of the stylet 776a, as best shown in FIGURE 17C. The distal end 752 of the catheter is returned to its neutral position (e.g., catheter non-deflected configuration) by translating the knob 784 away from the control handle 732, which in turn, retracts the bent distal end region 788a of the stylet 776a from the flexible distal section 746 to the proximal section 742, as shown in FIGURE 17B. To deflect the distal end of the catheter 730 in another direction, the knob 784 is rotated clockwise or counterclockwise to the desired position prior to advancing the stylet 776a into the catheter distal section 746. To that end, it will be appreciated that the stylet may be configured with high torque properties for turning the stylet 776a within the catheter 730. Thus, the distal end 752 of the catheter 730 may be deflected in any direction with respect to the longitudinal axis of the catheter. It will be appreciated that other mechanisms for advancing and retracting the stylet may be used, such as thumb slides or thumb wheels.

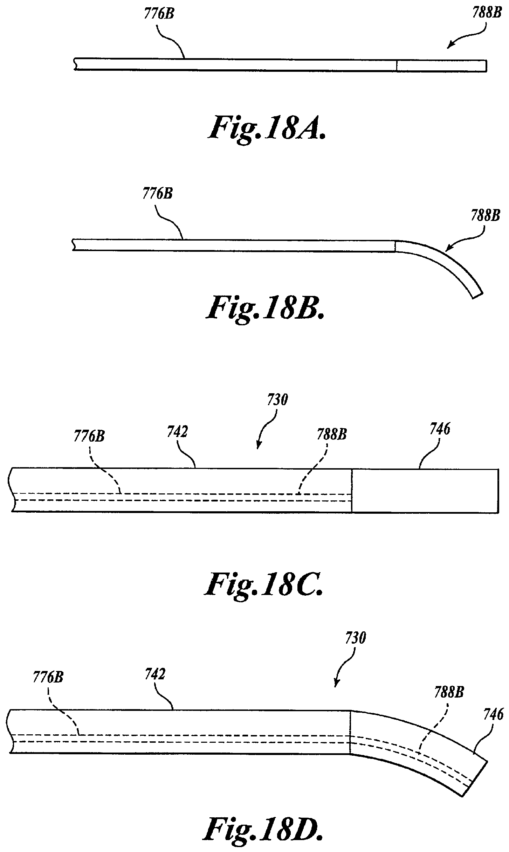

FIGURE 18A illustrates a partial view of another exemplary embodiment of a stylet 776b. In this embodiment, the stylet 776b may be constructed out of a shape memory material. The shape memory material preferably exhibits a memory characteristic in response to a condition change, such as temperature. In the illustrative embodiment, the shape memory material is a mechanical memory metal, such as a nickel titanium alloy. One nickel titanium alloy is commercially sold under the trademark Nitinol®. Shape-memory materials may be configured to have a first physical attribute, such as shape, under a first condition, such as a first temperature, and a second physical attribute, such as a different shape, under a second condition, such as a higher temperature. For example, the stylet 776b may be constructed such that it has a first shape (e.g., a straight configuration, as best shown in FIGURE 18A) when at a first temperature and a second shape (e.g., a configuration having a bent distal end region, as best shown in FIGURE 18B) when heated to a second higher temperature. While a nickel titanium alloy is desirable for construction of the stylet 776b, other materials having a memory characteristic relating to temperature or other conditions could be used.

In embodiments where a shape memory metal, such as nickel titanium, is used to construct the stylet 776b, the stylet 776b, in one example, can be annealed into its preformed shape (i.e., second shape) shown, for example, in FIGURE 18B. The stylet 776b is then cooled and straightened to its first shape shown, for example, in FIGURE 18A to allow for introduction into the catheter 730. In use, when the stylet 776b is again heated to or above a predetermined transitional (also known as transformational) temperature, the stylet 776b returns to its preformed shape shown in FIGURE 18B. As such, in this embodiment, the stylet 776b can be referred to as a temperature-activated stylet. In the illustrative embodiment, the predetermined transitional temperature may be any temperature above body temperature. For example, the predetermined transitional temperature may be in the range of approximately 100° to 150°F. In other embodiments, the transitional temperature of the stylet 776b may be close to but below normal patient temperatures. In either case, the stylet 776b is manufactured to attain the desired configuration when the temperature of the stylet 776b reaches the transitional temperature.

In this embodiment, the control handle 732 may further include a stylet heating system (not shown for ease of illustration) for increasing the temperature of the stylet 776b from a position external the body so as to deflect the distal end 752 of the catheter 730 in at least one desired direction corresponding to the preformed shape of the stylet 776b. In one embodiment, the stylet heating system includes a heating device connected in electrical communication with a power supply. The heating device is disposed within the control handle in heat transfer relationship with the stylet. In one embodiment, the heating device may be a positive thermal coefficient (PTC) heating element, which heats up when power is supplied thereto. The power supply may either be AC or DC, and can either be supplied to the control handle via a power chord or reside in the control handle as a power storage source, such as a battery. The stylet heating system further includes a control device, such as a switch, for selectively supplying power to the heating device. It will be appreciated that other various types of control devices may be employed.

In use, to deflect the distal end 752 of the catheter 730, the stylet 776b is first routed to the distal end 752 of the catheter 730 at a temperature below its transitional temperature (i.e., in its first, straight configuration shown in FIGURES 18A and 18C). The control switch is then activated, thereby supplying power to the heating device. Once the heating device receives power from the power source, the heating device increases in temperature and transfers its heat to the stylet 776b. Once the temperature of the stylet 776b increases above the transitional temperature, the stylet 776b retains its preformed shape, and accordingly, deflects the distal end 752 of the catheter 730 to the catheter deflected position shown in FIGURE 18D. To return the catheter 730 to its neutral (i.e., non-deflected) configuration shown in FIGURE 18C, the control switch is turned to its "off' position, thereby prohibiting the supply of power to the heating device. As such, the stylet 776b returns to its first temperature, which is below its transitional temperature. Once the stylet 776b attains a temperature below its transitional temperature, the stylet 776b straightens to its unbent position, which in turn, straightens the catheter 730 to the non-deflected configuration shown in FIGURE 18C.

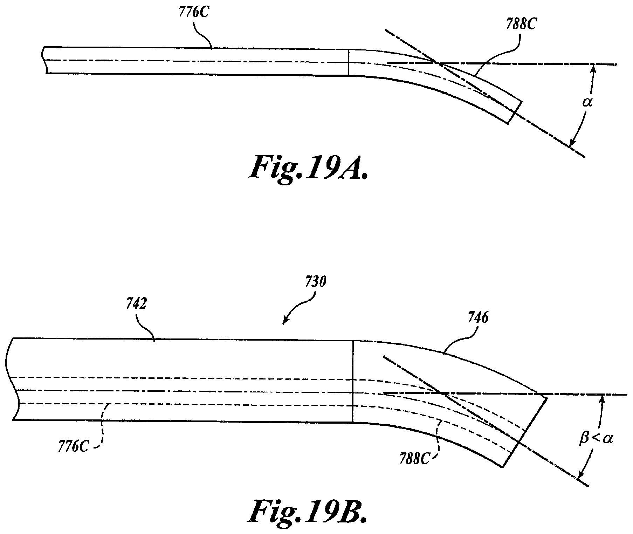

FIGURE 19A illustrates a partial view of another exemplary embodiment of a stylet 776c. stylet 776c is substantially similar in construction and operation as the stylet 776a except for the differences that will now be described in detail. In this embodiment, the stylet 776c may be constructed from stainless steel or other suitable biocompatible materials that are capable of being "overbent" to a given geometry. This overbending typically overcomes a problem associated with conventional materials, such as stainless steel, that occurs when the stylet is straightened for introduction. Typically, materials capable of being "overbent" are those metals or other materials that have elastic limits under approximately 2% and cannot return to their preformed geometries after straightening for introduction. Therefore, in this embodiment, the shape of the distal end region 788c of the stylet is exaggerated or "overbent", as shown in FIGURE 19A, so that it is capable of deflecting the catheter 730 into the desired geometries when introduced to the deflectable, distal section of the catheter. For example, the bent distal region 788c of the stylet 776c may attain an "overbent" deflection angle α of approximately 40 degrees, as shown in FIGURE 19A. Once straightened, introduced into the catheter, and routed to the end of the catheter, the stylet 776c returns to a deflection angle β less than the "overbent" deflection angle α, as shown in FIGURE 19B.

One exemplary method of using the catheter assembly 728 will now be described in detail. Prior to catheter introduction into the patient, one of the stylets 776a, 776b, or 776c may be loaded into the control handle 732 through the opening 780 and advanced into the steering device channel of the catheter 730. In embodiments that utilize the stylets 776a or 776c, the stylets are loaded by first straightening their bent distal end regions 788 against the restoring force generated by the material properties of the stylet and then advancing the straightened stylet into the distal section 742 of the catheter 730. In embodiments that utilize the stylet 776b, the stylet is advanced to the distal end 752 of the catheter 730. The catheter 730 may then be advanced through the selected passageways of the patient by moving the control handle 732 in order to reach the desired *in vivo* location. The catheter 730 may be advanced on its own or through a working channel of an endoscope. In embodiments that use the catheter assembly in conjunction with an endoscope, the control handle 732 is forwardly moved with respect to the biopsy port of the endoscope to advance the catheter 730.

As the catheter 730 is advanced through the passageways, it is sometimes desirable to deflect the distal end 752 of the catheter 730 to aid in locating and advancing the catheter to the desired *in vivo* location. To that end, in embodiments that utilize either the stylet 776a or 776c to deflect the distal end 752 of the catheter 730, the stylet is advanced by moving the knob 784 toward the handle 732 until the distal end region 788 of the stylet reaches the distal, more flexible section 746 of the catheter 730. Once the stylet reaches the distal section 746, the restoring force of the stylet overcomes the restraining force of the distal section 746, and, as a result, the distal end region of the stylet returns to its preformed configuration, thereby deflecting the distal section of the catheter 730 into the desired configuration. It will be appreciated that the user may alter the amount of deflection by the amount of stylet advancement within the distal section 746 of the catheter 730.

In embodiments that utilize the stylet 776b, the distal end 752 of the catheter 730 is deflected by activating the stylet 776b. To activate the stylet 776b, the temperature of the stylet is increased, for example, by a heating device that is capable of heating the stylet. Once the temperature of the stylet 776b increases above its transitional temperature, the stylet 776b retains its preformed shape, and, accordingly, deflects the distal end 746 of the catheter 730 into its desired configuration.

Once the catheter 730 is deflected in the desired direction, the catheter 730 is further advanced into the desired passageway. After the catheter 730 has entered the desired passageway, it may be desirable to return the distal end 752 of the catheter 730 to its neutral or non-deflected configuration. To that end, in the embodiment that utilizes the stylet 776a or 776c, the stylet is retracted by translating the knob 784 away from the control handle 732 so that the bent distal end region 788a of the stylet 776a moves from the flexible distal section 746 to the proximal section 742. Alternatively, in the embodiments that utilize the stylet 776B, the control switch is turned to its "off" position, thereby prohibiting the supply of power to the heating device. As such, the stylet 776b returns to its first temperature, which is below its transitional temperature. Once the stylet 776b attains a temperature below its transitional temperature, the stylet 776b straightens to its unbent configuration, which in turn, straightens the catheter to its neutral (i.e., non-deflected position) shown in FIGURE 18C. It will be appreciated that the body of the catheter 730 as the catheter is routed through the passageways may have a general curvature, and thus, it is contemplated that the catheter neutral configuration may include such general curvature.

Next, the distal end 752 of the catheter 730 can be alternatingly deflected and straightened so that the catheter 730 can advance to its desired *in vivo* position. In embodiments where it is desirable to deflect the distal end 752 of the catheter 730 in a direction different than the previous deflection, the stylets 776a-776c may be rotated within the steering device channel until the stylet is in an appropriate position to deflect the catheter in the desired direction. Once the catheter 730 has reached its desired *in vivo* position, instruments and/or viewing devices may be routed through respective catheter channels, as desired. Alternatively, the viewing device 1870 can be routed through the catheter 730 prior to or during catheter advancement for aiding in *in vivo* navigation through the passageways of the patient.

In an alternative method, the stylets 776a-776c can be loaded into the catheter 730 after the catheter has been inserted into the patient. This would allow the catheter 730 to be introduced quickly and easily in its straight configuration. This would also allow the physician to access the area of the body and decide on a stylet with the proper curve configuration without trying to guess ahead of time or rely on previous diagnoses.

Returning now to FIGURES 16A-16B, the control handle 732 may further include an attachment structure for selective attachment to an endoscope, if desired. In one embodiment, the catheter 730 is connected to the control handle via a connector fitting 770 that may act as, for example, a strain relief. As best shown in FIGURE 20, the connector fitting 770 may be configured in such a manner as to snugly fit into the biopsy port (BP) of an endoscope 124, and thus, functions as the attachment structure. When attached in this manner, the catheter 730 is aligned with the longitudinal axis of the biopsy port (BP). In an alternative embodiment, a rigid extension tube may be placed into the biopsy port, the free end of which receives the distal end of the connector fitting 770.

Alternative methods and configurations may be utilized for affecting selective deflection of the distal end of the catheters described herein. To that end, the following description includes several examples of control handles and/or steering mechanisms that may be utilized for deflecting the distal end of the catheter 130. Although exemplary embodiments of control handles/steering mechanisms may be described hereinafter for use with the catheter 130, it will be appreciated that aspects of the control handles/steering mechanisms hereinafter described have wide application, and may be suitable for use with catheters other than catheter 130, or other deflectable medical devices, including endoscopes, fiberscopes, steerable guidewires, etc. Accordingly, the following descriptions and referenced illustrations should be considered illustrative in nature.

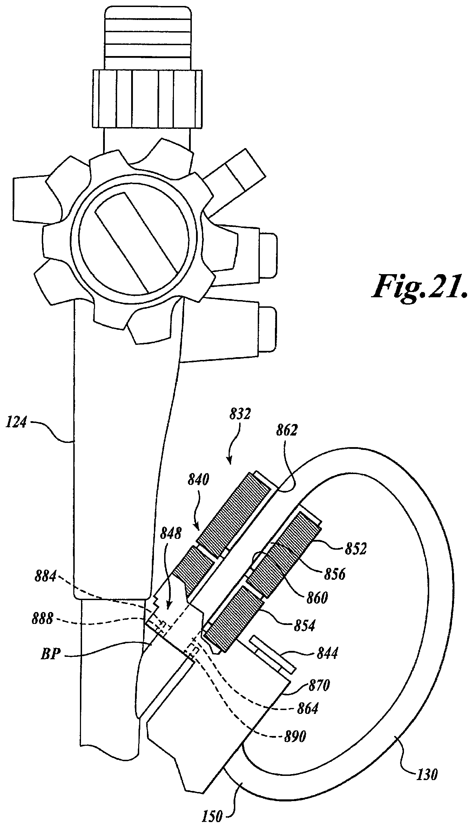

FIGURE 21 illustrates one embodiment of a control handle 832 that may be suitable for use with the catheter 130 or other conventional catheters that are deflected by one or more offset steering wires. In this embodiment, the control handle 832 may be selectively attached to the biopsy port (BP) of an endoscope 124, while concurrently or thereafter allowing a catheter 130 attached to the control handle 832 to be slideably routed through the endoscope's biopsy port (BP). As best shown in FIGURE 21, the control handle 832 includes a steering mechanism 840, at least one access port 844, and attachment structure 848 configured for selectively mounting the control handle 832 to the biopsy port or surrounding area of the endoscope, as will be described in more detail below.

As best shown in the partial cut-away view of the control handle 832, the steering mechanism 840 of the control handle 832 comprises first and second control knobs 852 and 854, respectively, rotatably retained to a central shaft 856. The central shaft 856 defines a longitudinal bore 860 having a longitudinal axis. The longitudinal bore 860 includes a first opening 862 at one end of the central shaft 856 for receiving the distal end of the catheter 130 and a second opening 864 at the opposite end for allowing the distal end of the catheter 130 to exit the control handle 832, as will be described in more detail below. The first and second control knobs 852 and 854 are mounted to the central shaft 856 for rotation about its longitudinal axis.

A base section 870 extends from the bottom of the central shaft 856. The base section 870 includes a third opening (hidden in FIGURE 21) to which the proximal end of the catheter 130 is selectively or permanently attached. The at least one access port 844 is positioned on the base section 870 for accessing one or more channels of the catheter 130. The base section 870 further includes suitable passageways (not shown) routed through the base section 870 to the control knobs 852 and 854. The passageways are sized and configured for freely routing the proximal ends of the steering wires (not shown) of the catheter 130 to the control knobs where the proximal ends of the steering wires are anchored to the control knobs in a conventional manner.

In accordance to one example, the control handle 832 further includes an attachment structure 848. In the embodiment shown, the attachment structure 848 is configured to selectively attach the control handle 832 to the biopsy port (BP) of the endoscope. In the embodiment shown, the attachment structure 848 is positioned such that when the control handle 832 is mounted to the endoscope, the longitudinal axis of the central shaft 856 is coaxial with the axis of the biopsy port (BP). In one embodiment, the attachment structure 848 may be comprised of a counterbore 884 concentrically arranged with the longitudinal bore 860 of the central shaft 856 and a resilient fitting member 888. The counterbore 884 communicates with and is larger than the second opening 864. The resilient fitting member 888, such as a rubber bushing, is mounted within the counterbore 884. The resilient fitting member 888 further includes a throughbore 890 sized and configured to be mounted over the biopsy port structure in a removably secure manner. The resilient fitting member throughbore 890 may include a lead-in to facilitate insertion of the biopsy port structure.

When assembled, the control handle 832 is detachably mounted on the biopsy port (BP) via the attachment structure 848. The catheter 130 extends from the base section 870, loops around to the first opening 862 of the control handle 832, and is inserted into the first opening 862. The catheter 130 is then routed through the longitudinal bore 860 of the central shaft 856 and exits the control handle 832 via the second opening 864. As will be described in more detail below, the distal end of the catheter 130 may exit off the second opening 864 and may be inserted into the biopsy port (BP) of the endoscope 124. First and second pairs of steering wires from the proximal end of the catheter 130 pass freely through the passageways of the base section 870, the proximal ends of which terminate at fixed connections to the first and second control knobs 852 and 854, respectively, in a conventional manner such that rotation of the first and second control knobs 852 and 854 selectively tension the first and second pairs of steering wires. In use, rotation of the first or second control knobs 852 and 854 selectively tension the steering wires, which in turn, deflects the distal end of the catheter 130 in one or more planes. Once the control handle 832 is mounted to the biopsy port (BP), the catheter 130 may concurrently be further advanced through the working channel of the endoscope by pushing the catheter by hand into the control handle first opening 862.

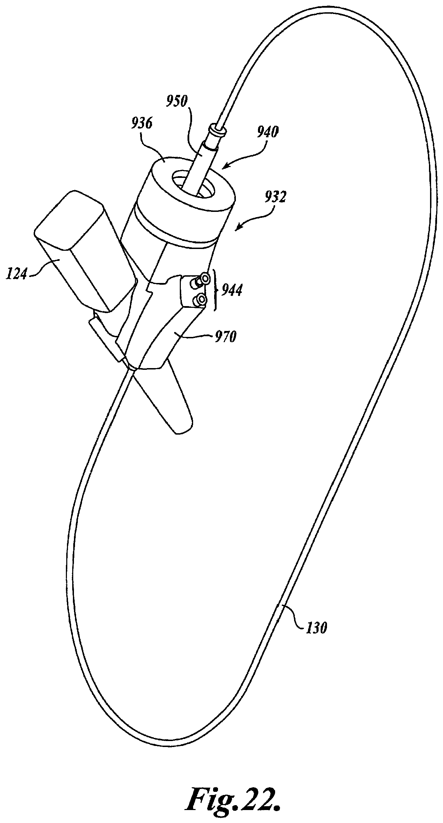

FIGURE 22 illustrates another embodiment of a control handle 932. The control handle 932 is substantially similar in construction, materials, and operation as the control handle shown in FIGURE 21, except for the differences that will now be explained. In this embodiment, the steering mechanism 940 of the control handle 932 is in the form of a joystick 950 instead of two rotational knobs. The first end of the joystick 950 is pivotally mounted within the handle housing 936 in a conventional manner, and preferably provides full 360° movement. The opposite end of the joystick 950 extends from the control handle 932, and is configured to be grasped by one hand of the user. The first and second pairs of the steering wires (not shown) extend from the proximal end of the catheter 130, are routed through the appropriate conduits of the base section, and terminate at a conventional connection with the first end of the joystick. As such, pivotal movement of the joystick 950 in one or more directions deflects the distal end of the catheter 130 via selective tensioning of the steering wires. The joystick 950 is further configured with a longitudinal bore (hidden in FIGURE 22) that is in communication with the counterbore of the attachment structure. The longitudinal bore defines the first and second openings (not shown) for catheter entrance into and exit from the control handle 932.

When assembled, the control handle 932 is detachably mounted on the biopsy port via the attachment structure. The catheter 130 extends from the base section 970, loops around to the first opening of the control handle 932 formed by the joystick 950, and is inserted into the first opening. The distal end of the catheter 130 is then routed through the longitudinal bore of the joystick 950 and exits the control handle 932 via the second opening. As will be described in more detail below, the catheter may exit off the second opening and may be inserted into the biopsy port (hidden in FIGURE 22) of the endoscope 124. First and second pairs of steering wires (not shown) from the proximal end of the catheter 130 pass freely through the passageways of the base section 970, the proximal ends of which terminate at fixed connections to the first end of the joystick 950 in a conventional manner such that pivoting the joystick 950 selectively tensions the first and second pairs of steering wires.

In use, pivoting of the joystick 950 selectively tensions the steering wires, which in turn, deflects the distal end of the catheter 130 in one or more planes. Once the control handle 932 is mounted to the biopsy port via the attachment structure (hidden in FIGURE 22), the catheter 130 may concurrently be further advanced through the working channel of the endoscope 124 by pushing the catheter 130 by hand into the first opening of the joystick 950. Specifically, with a single hand, the physician can advance and steer the catheter simultaneously. This may be accomplished by holding the catheter 130 just above the joystick 950. The catheter 130 can be advanced by axial force that pushes the distal end of the catheter 130 into the joystick 950 and may be simultaneously steered with lateral or pivoting movements of the joystick 950. A locking mechanism (not shown) may be optionally provided for keeping the joystick 950 in the selected position, if desired.

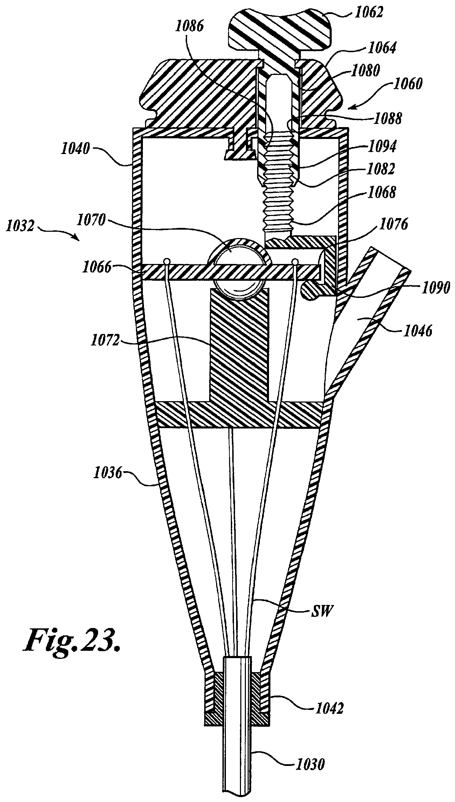

FIGURE 23 illustrates another embodiment of a control handle 1032 capable of effecting deflection of the distal end of a catheter 1030. The control handle 1032 includes a handle housing 1036 having a proximal end 1040 and a distal end 1042. The distal end 1042 of the handle housing is functionally connectable to the proximal end of the catheter 1030. The control handle 1032 may include one or more access ports 1046 that communicate with one or more channels of the catheter 1030. The ports 1046 are connected in communication with the catheter channels through one or more conduits (not shown) that route through the handle housing 1036.

The control handle 1032 further includes a steering mechanism 1060 for deflecting the distal end of the catheter in one or more planes. In the embodiment shown, the steering mechanism 1060 includes first and second knobs 1062 and 1064, a circular swash plate 1066, and mechanical linkage 1068 interconnecting the circular swash plate 1066 with the first and second knobs 1062 and 1064. The circular swash plate 1066 is mounted within the handle housing 1036 on a central pivot 1070 substantially aligned with the proximal end of the catheter. The central pivot 1070 is a ball-like structure that is supported by a pivot base 1072. The proximal ends of the catheter steering wires (SW) pass through the handle housing 1036 and are connected around the circumference of the circular swash plate 1066 at equidistant locations spaced radially inward from the outer perimeter edge 1076, as shown. For example, in a four-wire configuration, the wires are connected to the circular swash plate 1066 in 90° intervals. In a three-wire configuration, the steering wires are connected to the circular swash plate 1066 in 120° intervals.

The second knob 1064 is rotatably mounted to the handle housing 1036 at a position such that its axis of rotation is coaxial with the central pivot 1070. The second knob 1064 defines a cylindrical throughbore 1080 offset from its rotational axis. The throughbore 1080 of the second knob 1064 rotatably receives a first knob shaft 1082 of the first knob 1062. When assembled, the first knob shaft of the first knob 1062 extends through the throughbore 1080 of the second knob 1064 and into the handle housing 1036. As such, the first knob 1062 is rotatably supported by the second knob 1064. The handle housing 1036 includes a circular slot 1088 through which the first knob shaft 1082 extends, the reasons for which will be described in detail below. The first knob shaft 1082 defines an internally threaded bore 1086.

As shown in FIGURE 23, the mechanical linkage 1068 interconnects the first and second knobs 1062 and 1064 and the swash plate 1066 for transmitting rotational movement of the knobs into pivotal movement of the swash plate 1066, and in turn, translational movement of the steering wires (SW). In the embodiment shown in FIGURE 23, the mechanical linkage 1068 includes a hook 1090 at one end that rides under the outer perimeter edge 1076 of the circular swash plate 1066 and a lead screw 1094 at its opposite end threadedly coupled to the internally threaded bore 1086 of the first knob shaft 1082. Accordingly, rotation of the first knob 1062 causes the mechanical linkage to linearly translate within the internally threaded bore of the first knob shaft 1082. Movement, e.g., upward translation, of the mechanical linkage 1068 causes the hook 1090 to contact the circular swash plate 1066, which in turn, causes the circular swash plate 1066 to pivot relative to the central pivot 1070. As the swash plate 1066 pivots about the central pivot 1070, the swash plate 1066 pulls one or more of the steering wires (SW) and causes the distal end of the catheter 1030 to deflect in the desired direction.

To deflect the distal end of the catheter 1030 in a different direction, the second knob 1064 is rotated either in a clockwise or counterclockwise direction, depending on the desired deflection direction. Rotation of the second knob 1064 causes the first knob 1062 to rotate around the axis of the second knob 1064 through the circular slot 1088 of the handle housing 1036. As a result, the hook 1090 rotates around the perimeter of the circular swash plate 1066. Once the hook 1090 has attained the desired position, the first knob 1062 may then be rotated as previously described to alter the pivot angle of the swash plate 1066, and thus, the deflection angle of the catheter distal end.

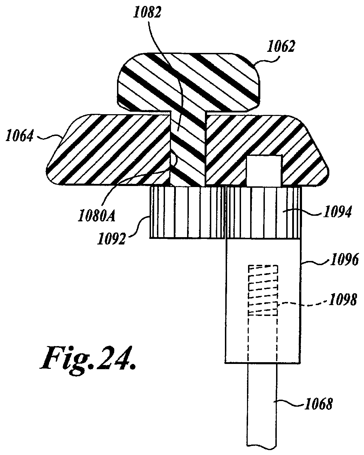

In an alternative embodiment, both first and second knobs 1062 and 1064 may be mounted on-axis with the central pivot. In this embodiment shown in FIGURE 24, the shaft 1082 of the first knob 1062 is rotatably received within a central bore 1080A of the second knob 1064. The shaft 1082 of the first knob 1062 extends past the second knob 1064, and terminates as a gear 1092. The gear 1092 meshingly engages gear teeth 1094 located around a second shaft 1096, which is journaled for rotation to the bottom of the second knob 1064 on the handle housing. The second shaft 1096 defines an internally threaded bore 1098 to which the mechanical linkage 1068 is threadably engaged. Thus, when the second knob 1064 is rotated, the second shaft 1096 rotates around the gear 1092 of the first knob 1062. When the first knob 1062 is rotated, the gear 1092 of the first knob 1062 rotates the geared shaft 1096, and thus, linearly translates the mechanical linkage 1068, causing the circular swash plate to tilt, thereby deflecting the distal end of the catheter.

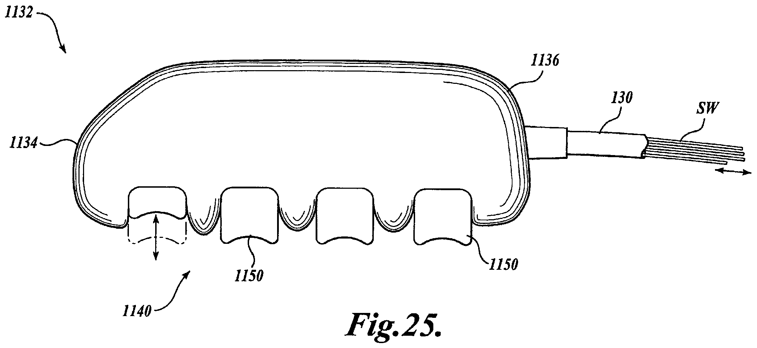

FIGURE 25 illustrates another embodiment of a control handle 1132 for deflecting the distal end of an associated catheter 130. The control handle 1132 defines a proximal end 1134 and a distal end 1136 to which the proximal end of the catheter is functionally connected. The handle 1132 further includes a steering mechanism 1140 in the form of one or more depressible buttons 1150 that can separately actuate one or more steering wires (SW) to deflect the distal end of the catheter 130. It will be appreciated that the number of depressible buttons 1150 corresponds to the number of steering wires (SW). For example, a catheter having two pairs of steering wires will be connected to a handle having four depressible buttons, a catheter having three steering wires will be connected to a handle having three depressible buttons, and so on. The control handle 1132 is preferably ergonomically configured to be grasped by the physician's hand, and the placement of the buttons 1150 is such that the buttons 1150 may be depressed by the physician's fingers.

To facilitate identification of the buttons 1150, the top of each depressible button 1150 may include a depression, a groove, or a raised structure. In the embodiment shown, the depressible buttons 1150 are depressed or actuated along axes that are perpendicular to the longitudinal axis of the catheter 130; however, the catheter may be positioned such that the longitudinal axis of the catheter 130 is substantially parallel with the axis of travel of the depressible buttons 1150. It will be appreciated that any mechanical linkage that transmits the movement of the buttons 1150 into tension on the steering wires may be used, such as rocker arms, levers, cranks, or combinations thereof. Access to the channels of the catheter 130 may be provided by access ports (not shown) positioned on the handle or via a breakout box or other structure attached to the catheter separate from the handle.

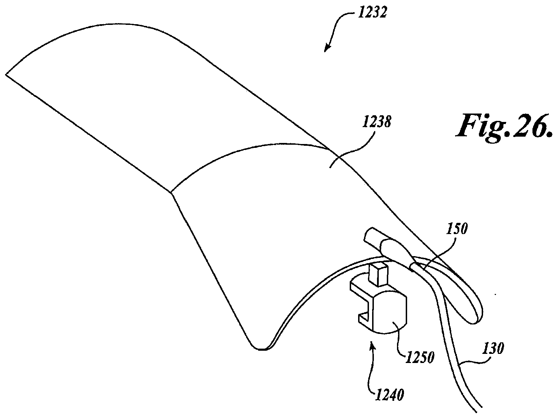

FIGURE 26 illustrates another embodiment of a control handle 1232 for deflecting the distal end of an associated catheter 130. In this embodiment, the control handle 1232 is configured to be mounted over the forearm and hand of the user, such as a physician. The control handle 1232 includes a curved surface 1238 that is suitably dimensioned to be mounted on a physician's forearm and hand. The control handle 1232 defines at one end an opening (hidden by the catheter in FIGURE 26) to which the proximal end of the catheter 130 is connected. The control handle 1232 also includes one or more access ports (not shown) that communicate with the channels of the catheter 130 through one or more passageways. The control handle 1232 further includes a steering mechanism 1240 for deflecting the distal end of the catheter 130. In the embodiment shown, the steering mechanism 1240 includes a cap-like structure 1250 that may attach to one of the physician's fingers. The cap-like structure 1250 is connected to one or more of the steering wires (SW) of the catheter 130. Thus, the distal end of the catheter 130 is steered via movement of the physician's finger in a direction that would apply tension on the steering wire (SW). It is envisioned that the motion of the physician's hand when performing functions, such as manipulating the endoscope, using biopsy forceps, etc., would not interfere with the physician's ability to steer/maintain position of the catheter.

It may be desirable to configure a control handle control feature, such as the steering mechanism, to multiply the input movement of a steering input device (e.g., steering knob, slides, dials, joystick, etc.), the movement of which is used to control the deflection of the distal end of a catheter. By multiplying the input distance of the input device, larger axial movement of the steering wires is achieved, and as a result, a larger deflection of the catheter distal end. Larger deflection of the distal end of the catheter from smaller movement of the input device may provide many advantages; for example, it potentially allows for the construction of smaller steering mechanisms, which in turn, may allow for smaller handles for medical devices (e.g., catheter, endoscope, etc., or other control handle). To that end, several exemplary embodiments of steering mechanisms suitable for use with control handles, such as medical device handles, are described in detail below for multiplying the movement of the steering input device, and thus, achieving larger catheter distal end deflection.

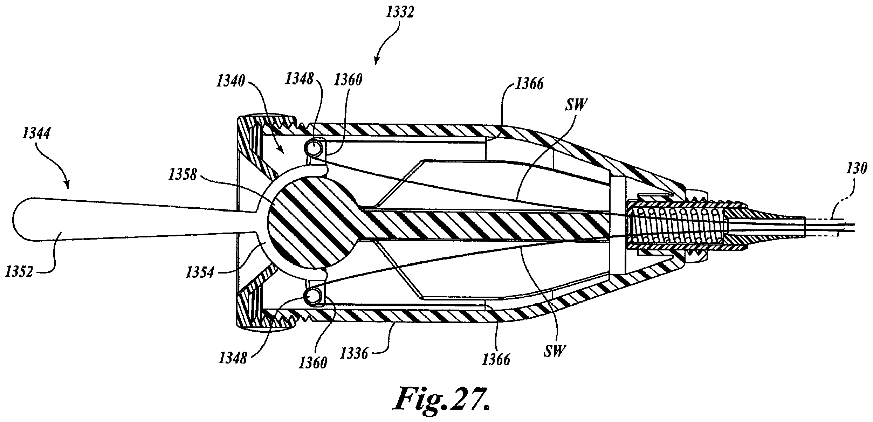

FIGURE 27 illustrates one embodiment of a control handle 1332 adapted to be functionally connected to a catheter, such as catheter 130, having one or more steering wires (SW) anchored at its distal end. The control handle 1332 includes a handle housing 1336 to which an exemplary steering mechanism 1340 is operationally mounted. The steering mechanism 1340 of the control handle 1332 is configured for deflecting the distal end of the catheter 130 via selective axial translation of the steering wires (SW) of the catheter 130. The steering mechanism 1340 comprises a steering input device 1344 and one or more movement multiplying devices 1348. The steering input device 1344 is coupled to a portion of each steering wire (SW) via the movement multiplying devices 1348. As such, axial translation of one or more steering wires (SW) is effected by movement of the steering input device 1344 through one or more movement multiplying devices 1348. As will be described in more detail below, the movement multiplying devices 1348 multiply the movement (i.e., translation distance, referred to sometimes herein as the stroke) of the steering input device 1344, resulting in larger axial movement of the steering wires (SW), and thus, larger deflection angles of the distal end of the catheter.

In the embodiment shown in FIGURE 27, the steering input device 1344 is a joystick having an elongated portion 1352 at its first end and a semi-circular swash plate 1354 at its second end. The semi-circular swash plate 1354 of the joystick is pivotally mounted to the handle housing on a central pivot 1358, such as a ball-like structure. The semi-circular swash plate 1354 is capable of pivoting up to 360 degrees on the central pivot. Around the outer periphery of the swash plate 1354, there is provided a plurality of attachment flanges 1360 on which a plurality of movement multiplying devices 1348, respectively, are supportively mounted. In this embodiment, the movement multiplying devices 1348 are pulleys (hereinafter referred to as pulley(s) 1348), one pulley 1348 being supportively mounted on each attachment flange. Each pulley 1348 is mounted for rotation on each attachment flange 1360, and defines a rotational axis substantially perpendicular to the longitudinal axis of the catheter 130 at the connection of the catheter to the distal end of the control handle 1332.

When assembled, the proximal ends of the steering wires (SW) are routed from the proximal end of the catheter 130 over the pulleys 1348 and back toward the proximal end of the catheter 130 where they are anchored at fixed positions 1366 within the interior of the handle housing 1336. In use, the elongated portion 1352 of the joystick may be grasped by the user and pivoted about the central pivot 1358, thereby moving one or more of the attachment flanges 1360, which in turn, moves one or more of the respective pulleys 1348. Movement of the pulleys 1348 tension one or more of the steering wires (SW) and axially translates the steering wires (SW) for deflecting the distal end of the catheter 130.

In accordance with engineering mechanics, it will be appreciated that the use of the moving pulleys 1348 in the manner shown and described for interconnecting the steering wires (SW) with the input device 1344 (e.g., joystick) multiplies the movement of the input device 1344 (as measured at the attachment flange) by a multiplication factor (in this case the multiplication factor equals two (2)), resulting in larger axial movement of the steering wires (SW) as compared with attaching the proximal ends of the steering wires directly to the attachment flanges without the pulleys. It will be appreciated that additionally pulleys may be employed and arranged using routine skill in the art to further increase the multiplication effect.

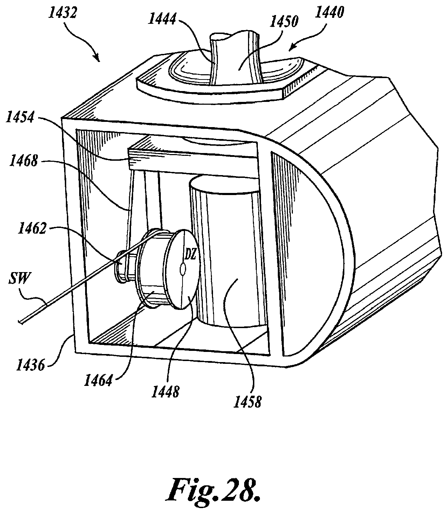

FIGURE 28 illustrates a partial view of another embodiment of a control handle 1432 that employs a steering mechanism 1440 that multiplies the input movement of the input device for deflecting the distal end of the catheter. The control handle 1432 is substantially similar in construction, materials, and operation as the control handle shown in FIGURE 27, except for the differences that will now be explained. The control handle 1432 includes a handle housing 1436 to which the exemplary steering mechanism 1440 is operationally mounted. The steering mechanism 1440 of the control handle comprises a steering input device 1444 and one or more movement multiplying devices 1448. In the embodiment shown, the input device 1444 is a joystick 1450 and the movement multiplying devices 1448 are one or more spools (hereinafter referred to as spools 1448). For ease of illustration, only one spool is shown, however; it will be appreciated that one spool corresponds to one steering wire of the catheter. Thus, in embodiments that use a four-steering-wire catheter, the control handle includes four spools spaced 90 degrees apart. The joystick 1450 includes a graspable shaft portion at one end and a swash plate 1454 at the other. The joystick 1450 is pivotally mounted at the swash plate 1454 to a central pivot 1458 fixed in the handle housing 1436. The swash plate 1454 includes flange members 1460 (hidden from view), the corners of which are connected to respective spools 1448 via wires 1468, as will be described in more detail below.

The spools 1448 are rotatably mounted to the handle housing 1436. Each spool includes first and second spool sections 1462 and 1464 having diameters D1 and D2, respectively. In this embodiment, the diameter D2 is greater than diameter D1. The wires 1468 that connect the swash plate 1454 to the spools 1448 are wound around the smaller diameter first spool sections 1462. The proximal end of the steering wires (SW) of the catheter (not shown) are wound partially around the larger diameter second spool sections 1464 and fixedly connected thereto.

According to engineering mechanics, the spools 1448 act like a wheel and axle mechanism to multiply the input device movement by a multiplication factor determined by the diameters of D1 and D2. Specifically, by applying the effort force via the input device 1444 to the outer periphery of the smaller diameter first spool section 1462 (i.e., the axle) and by applying the resistance force from the steering wires SW to the outer periphery of the larger diameter second spool section 1464 (i.e., the wheel), the distance of the input device 1444 is multiplied by the ratio of D2:D1 (i.e., the multiplication factor is the ratio of D2:D1). Accordingly, smaller movement of the joystick 1450 effects larger deflection of the distal end of the catheter. It will be appreciated that the ratio of diameter D2 to diameter D1 may be changed to increase/decrease the movement of the steering wires.

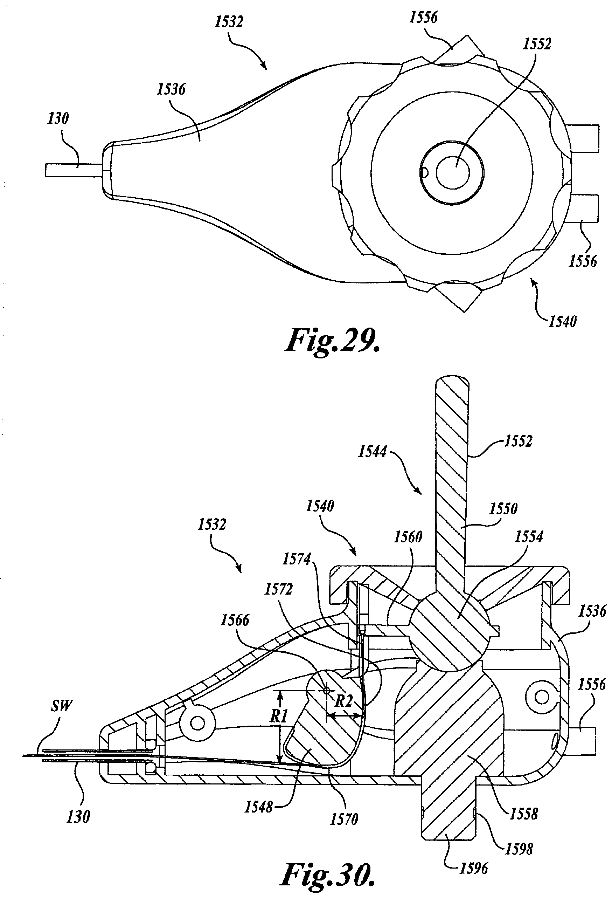

FIGURES 29-31 illustrate another embodiment of a control handle 1532 that employs a steering mechanism 1540 that multiplies the input movement of the input device for deflecting the distal end of the catheter. The control handle 1532 is substantially similar in construction, materials, and operation as control handles shown in FIGURES 27 and 28, except for the differences that will know be explained. The control handle 1532 includes a handle housing 1536 to which the exemplary steering mechanism 1540 is operationally mounted. The steering mechanism 1540 of the control handle comprises a steering input device 1544 and one or more movement multiplying devices 1448.

In this embodiment, the input device 1544 is a joystick 1550 and the movement multiplying devices 1448 are one or more bell cranks (hereinafter referred to as bell cranks 1548). For ease of illustration, only one bell crank is shown, however; it will be appreciated that one bell crank corresponds to one steering wire (SW) of the catheter 130. Thus, in embodiments that use a four-steering-wire catheter, the control handle 1532 includes four bell cranks 1548 spaced 90 degrees apart. The joystick 1550 includes a graspable shaft portion 1552 at one end and a ball-like member 1554 at the other. The joystick 1550 is pivotally mounted at the ball-like member 1554 to a fixed support 1558 defined by or coupled to the handle housing 1536. The joystick 1550 includes one or more flange members 1560 integrally formed with the ball-like member and laterally extending therefrom. The number of flange members 1560 corresponds to the number of steering wires (SW), and thus, the number of bell cranks 1548.

Each bell crank 1548 is pivotally mounted within the handle housing 1536 about a pivoting axis 1566 that is disposed perpendicular to the longitudinal axis of the steering wires. Each steering wire (SW) of the catheter 130 is connected to a corresponding bell crank 1548 at a first connection 1570 that is spaced a radial distance R1 from the pivoting axis 1566. The bell crank 1548 is linked at a second connection 1572 that is spaced a distance R2 from the pivoting axis 1566 to a corresponding flange member 1560 of the joystick 1550 via a linkage 1574. The linkage 1574 may be a flexible linkage, such as a wire, or a rigid linkage, such as a link or bar. As such, the bell cranks 1548 connect the steering wires (SW) to the joystick 1550.

In use, the joystick 1550 may be grasped by the physician and pivoted in one or more directions, thereby axially moving one or more of the steering wires (SW) to effect distal end deflection of the catheter 130. As the joystick 1550 pivots, the flange members 1560 integrally formed therewith also pivot, thereby defining an input distance or stroke. The movement of the flange members 1560 through the input stroke applies an effort force on the linkage 1574 and translates the linkage 1574 a selected distance. The translation of the linkage 1574, in turn, rotates the bell crank 1548 in a counterclockwise direction as shown in FIGURE 30 about its pivoting axis 1566. When the bell crank 1548 rotates, it tensions the respective steering wire (SW) due to the connection of the steering wire (SW) to the bell crank 1548, and translates the steering wire (SW) away from the distal end of the catheter 130. The translation of the steering wire (SW) deflects the distal end of the catheter 130 in the chosen direction. As such, the steering wire (SW) places a resistance force against the bell crank 1548.

According to engineering mechanics, by connecting the steering wires (SW) to the input device 1544, i.e., the joystick 1550, via the bell cranks 1548 in the manner shown and described, the bell cranks 1548 multiply the input distance or stroke of the joystick 1550 by a multiplication factor determined by R1 and R2 for achieving larger axial movement of the steering wires (SW). Specifically, by applying the effort force via the input device 1544 on the bell cranks 1548 at a distance R2 from the pivoting axis 1566 and by applying the resistance force from the steering wires (SW) on the bell cranks 1548 at a distance R1 from the pivoting axis 1566, the input distance or stroke of the input device 1544 is multiplied by the ratio of R1:R2 (i.e., the multiplication factor is the ratio of R1:R2). Since the distance R1 between the first connection 1570 and the pivoting axis 1566 of the bell crank 1548 is greater than the distance R2 between the second connection 1572 and the pivoting axis 1566 of the bell crank 1548, the bell cranks 1548 multiply the stroke of the input device by a ratio, i.e., the multiplication factor, that is greater than one. It will be appreciated that one could change the ratio of R1:R2 to effect greater or lesser steering wire movement.

The control handles described above with reference to FIGURES 27-31 may include other features, as will now be described. The control handles may include one or more access ports, such as the ports 1556 shown in FIGURES 29-31. The access ports, such as access ports 1556, are connected in communication with one of more channels provided in the catheter, thereby providing access from outside the control handle to the distal end of the catheter via the catheter channels. For example, the access ports 1556 may be connected to the working channel, the irrigation channel, and/or viewing device channel of an appropriately configured catheter.

The control handles may optionally include attachment structure for selectively attaching the control handles to an associated endoscope, or other structure, such as a surgical cart, etc. The attachment structure may be integrally formed with the handle or may be a discrete device or assembly that attaches the control handle to the associated structure. The attachment structure can be any structure capable of selectively attaching the control handle to the desired object. Such attachment structure may include straps, clamps, clamshell-type connectors, brackets, etc., and may depend on the object to which the control handle is attached. For example, a clamp may be more suitable for attachment to a surgical chart or the like, while straps, brackets, or the like, may be more suitable for attachment onto an endoscope of other medical devices.

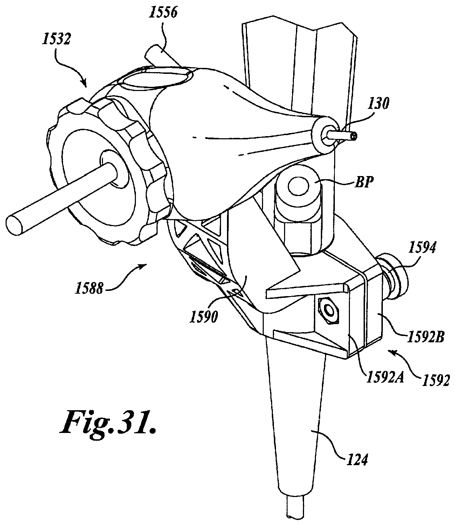

In the embodiment shown in FIGURES 30 and 31, the control handle 1532 is selectively attached to the endoscope 124 via an attachment structure 1588. As best shown in FIGURE 31, the attachment structure 1588 includes an armature 1590 integrally formed with a bracket 1592 of the clamshell type. The bracket 1592 is configured for selective attachment around a portion of the endoscope body, as shown in FIGURE 31. The bracket 1592 includes left and right bracket halves 1592a and 1592b that envelop the body of the endoscope 124. The bracket halves 1592a and 1592b are pressed together at respective ends via fasteners 1594, such as bolts, for securely connecting the attachment structure 1588 to the endoscope 124. The armature 1590 includes a distal end portion (hidden by the control handle 1532 in FIGURE 31) that defines a control handle connection interface (hidden by the control handle 1532) for selective attachment to the control handle 1532.

In one embodiment, the control handle interface is cooperatively configured for receiving an attachment projection 1596 (see FIGURE 30) of the control handle 1532 in a manner that permits the control handle 1532 to adjustably rotate with respect to the attachment structure 1588 between preselected fixed positions. For example, in one embodiment, the attachment projection 1596 may be formed with detents 1598 that suitably cooperate with the control handle connection interface for forming an indexing mechanism. As such, the rotational movement of the control handle 1532 may be indexed between fixed positions with respect to the attachment structure 1588. Accordingly, the control handle 1532 may have many orientations with respect to the endoscope 124 when selectively mounted thereto. For example, the longitudinal axis of the control handle 1532 may be perpendicular to the central axis of the endoscope biopsy port (BP), or may form any desirable acute or obtuse angle with respect to the central axis of the endoscope biopsy port (BP).

A catheter assembly can be mounted to the endoscope handle so that the physician can manipulate both the endoscope and the catheter assembly using two hands. As a result of connecting the catheter assembly to the endoscope, as shown in FIGURES 1, 21, 22, and 31, the catheter 130 creates a loop, known as a service loop, prior to entrance into the biopsy port (BP). In some instances, this may create binding or friction of the instruments, wires, etc., as they slideably move through the catheter during use. To this end, several exemplary configurations may be employed to ease such potential binding, as will now be described in detail.

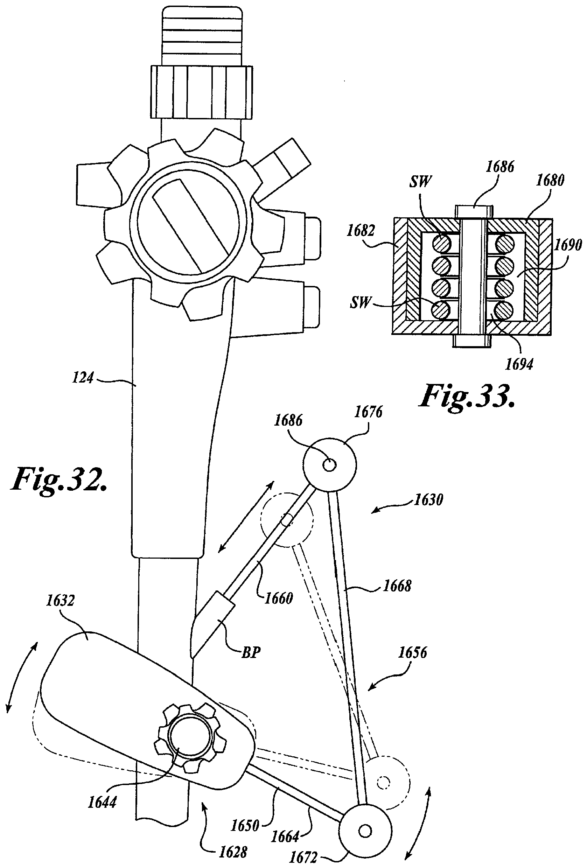

Turning now to FIGURE 32, there is shown one exemplary embodiment of a catheter assembly 1628 comprised of a catheter 1630 and a control handle 1632 mounted to an associated endoscope 124 for use therewith. In the embodiment shown, the catheter 1630 employs a particular configuration that aids in the reduction of catheter binding and helps guide the distal end of the catheter 1630 into the biopsy port (BP) of the associated endoscope 124. The control handle 1632 includes an input steering device 1644 for controlling the deflecting of the catheter. The catheter 1630 defines a proximal end 1650 and a distal end. The proximal end 1650 of the catheter 1630 is functionally connected to the control handle 1632. The catheter 1630 further includes one or more steering wires (SW) that are anchored at the distal end of the catheter 1630 and extend past the proximal end 1650 of the catheter 1630 for connection to the steering input device 1644. The steering wires (SW) are movable within the catheter 1630 as a result of activation of the steering input device 1644 for deflecting the distal end of the catheter 1630.

In one embodiment, the catheter 1630 is formed with a deflectable distal section (hidden by the endoscope in FIGURE 32) and a proximal section 1656. In the embodiment shown, the proximal section 1656 comprises a flexible segment 1660 and first and second semi-rigid or rigid segments 1664 and 1668 hingedly connected, as shown schematically in FIGURE 33. In this embodiment, the first and second semi-rigid or rigid segments 1664 and 1668 and the flexible segment 1660 of the proximal section are interconnected by first and second hinge mechanisms 1672 and 1676. The hinge mechanisms 1672 and 1676 each include top and bottom sections 1680 and 1682 hingedly connected to one another via a central cylindrical shaft 1686, as best shown in the cross-sectional view of FIGURE 33.

The first semi-rigid or rigid section 1664 is attached to either the top or the bottom section of the first hinge mechanism 1672 and the second semi-rigid or rigid section 1668 is connected to the other of the top or bottom section of the first hinge mechanism 1672. Similarly, the second semi-rigid or rigid section 1668 is attached to either the top or the bottom section of the second hinge mechanism 1676 and the flexible segment 1660 is connected to the other of the top or bottom section of the second hinge mechanism 1676. When assembled, the top and bottom sections of the hinge assemblies 1672 and 1676 define an inner cavity 1690. The hinge assemblies 1672 and 1676 further include a set of pulleys 1694 rotationally mounted to the central shaft within the inner cavity 1690. The pulleys 1694 are configured so as to redirect the steering wires SW as they traverse through the catheter 1630.

In use, the control handle 1632 is mounted to the endoscope 124 in a manner such that the catheter 1630 extends at an angle from the axis of the biopsy port (BP). In the embodiment shown, the control handle 1632 is rotatably mounted to the endoscope 124; however, in other embodiments it is possible for the control handle 1632 to be securely attached to the endoscope 124 in a fixed position. When the distal end of the catheter 1630 is advanced into the biopsy port (BP) of the associated endoscope 124, the segments 1660, 1664, and 1668 rotate with respect to one another, allowing the longitudinal axis of the distal section and the flexible segment 1660 to remain substantial coaxial with the central axis of the biopsy port BP as the catheter is guided into the biopsy port (BP).

The invention is illustrated in Fig.34. There is shown a catheter assembly 1728 mounted to an associated endoscope 124 for use therewith. The catheter assembly 1728 according to the invention employs a particular configuration that aids in the reduction of catheter binding and helps guide the distal end of the catheter into the biopsy port of the associated endoscope. As best shown in FIGURE 34, the catheter assembly 1728 is movably connected to the endoscope 124 via a slide bar mechanism 1740. The slide bar mechanism 1740 is either permanently or removably attached to the endoscope 124. The slide bar mechanism 1740 includes a bracket 1748 at one end for connecting the slide bar mechanism 1740 to the endoscope 124. The slide bar mechanism 1740 further includes an elongate member 1750 attached to the bracket 1748. The slide bar member 1750 extends outwardly from the endoscope 124 when attached hereto, and extends parallel with the central axis of the biopsy port BP.

In this embodiment of the invention, the control handle 1732 includes a lateral extension 1760 having a throughbore 1762 through which the slide bar member 1750 is received. The throughbore 1762 is positioned such that when routed over the slide bar member 1750, the catheter 130 is in general alignment with the biopsy port (BP). In use, as the catheter 130 is advanced through the biopsy port (BP), the lateral extension 1760 of the control handle 1732 is routed over the slide bar member 1750 so that the slide bar member 1750 guides the insertion of the catheter 130 into the biopsy port (BP). As such, by aligning the catheter 130 with the central axis of the biopsy port (BP), the slide bar mechanism 1740 aids in the insertion of the catheter 130 and reduces binding at the proximal section of the catheter.

The principles, exemplary embodiments, and modes of operation have been described in the foregoing description.

The embodiments described herein are to be regarded as illustrative rather than restrictive. Variations and changes may be made by others, and equivalents employed.

## Claims

1. A catheter assembly (1728) for mounting a catheter (130) to an endoscopic device (124), comprising:
a slide bar mechanism (1740) which is attached to the endoscopic device (124) and is configured to guide insertion of the catheter (130) into the endoscopic device (124);
an elongate slide bar member (1750) extending outward from the endoscopic device (124) and extending parallel to a central axis of a port of the endoscopic device (124); and
a lateral extension (1760), which, during use, extends between the elongate slide bar member (1750) and
the catheter (130) to generally align the catheter (130) with the port to permit insertion of the catheter (130) into the port, **characterized in that**
the lateral extension (1760) is configured to be routed over the elongate slide bar member (1750) so that the elongate slide bar member (1750) guides the insertion of the catheter (130) into the port.

2. The assembly of Claim 1, wherein the slide bar mechanism (1740) is permanently attached to the endoscopic device (124).

3. The assembly of Claim 1, wherein the slide bar mechanism (1740) is removably attached to the endoscopic device (124).

4. The assembly of Claim 1, wherein the slide bar mechanism (1740) includes a bracket (1748) configured to connect to the endoscopic device (124).

5. The assembly of Claim 4, wherein the bracket (1748) is attached to the elongate slide bar member (1750).

6. The assembly of Claim 1, wherein the lateral extension (1760) comprises a throughbore configured to receive the elongate slide bar member (1750).

7. The assembly of Claim 1, wherein a control handle (1732) of the catheter (130) includes the extension.

8. The assembly of Claim 7, wherein the control handle (1732) includes a steering mechanism for controlling the deflection of a distal end of the catheter (130).

9. The assembly of Claim 8, wherein the steering mechanism includes a steering input device coupled to one or more steering members.

10. The assembly of Claim 1, wherein the port includes a biopsy port.

11. The assembly of Claim 1, further including the catheter.

12. The assembly of any of claims 1 to 10 and additionally comprising at least one of the following features:
the catheter (130) having a proximal end and a distal end;
a steering wire attached to the distal end of the catheter (130) and being axially movable relative to the catheter to cause the distal end of the catheter to deflect in at least one direction;
a handle housing functionally connected to the proximal end of the catheter at a connection interface, the proximal end of the at least one steering wire passing into the handle housing; and
a first knob rotationally supported by the handle housing.

## Patentansprüche

1. Katheteranordnung (1728) zum Anbringen eines Katheters (130) an einer Endoskopvorrichtung (124), die aufweist:
einen Gleitschienenmechanismus (1740), der an der Endoskopvorrichtung (124) angebracht und so konfiguriert ist, dass er das Einführen des Katheters (130) in die Endoskopvorrichtung (124) führt;
ein längliches Gleitschienenteil (1750), das sich von der Endoskopvorrichtung (124) nach außen erstreckt und sich parallel zu einer Mittelachse eines Ports der Endoskopvorrichtung (124) erstreckt; und
eine Querverlängerung (1760), die sich im Gebrauch zwischen dem länglichen Gleitschienenteil (1750) und dem Katheter (130) erstreckt, um den Katheter (130) allgemein zum Port auszurichten und das Einführen des Katheters (130) in den Port zu ermöglichen, **dadurch gekennzeichnet, dass**
die Querverlängerung (1760) so konfiguriert ist, dass sie über das längliche Gleitschienenteil (1750) geführt ist, so dass das längliche Gleitschienenteil (1750) das Einführen des Katheters (130) in den Port führt.

2. Anordnung nach Anspruch 1, wobei der Gleitschienenmechanismus (1740) an der Endoskopvorrichtung (124) dauerhaft angebracht ist.

3. Anordnung nach Anspruch 1, wobei der Gleitschienenmechanismus (1740) an der Endoskopvorrichtung (124) entfernbar angebracht ist.

4. Anordnung nach Anspruch 1, wobei der Gleitschienenmechanismus (1740) eine Halterung (1748) aufweist, die so konfiguriert ist, dass sie mit der Endoskopvorrichtung (124) verbunden ist.

5. Anordnung nach Anspruch 4, wobei die Halterung (1748) am länglichen Gleitschienenteil (1750) angebracht ist.

6. Anordnung nach Anspruch 1, wobei die Querverlängerung (1760) eine Durchgangsbohrung aufweist, die so konfiguriert ist, dass sie das längliche Gleitschienenteil (1750) aufnimmt.

7. Anordnung nach Anspruch 1, wobei ein Steuergriff (1732) des Katheters (130) die Verlängerung aufweist.

8. Anordnung nach Anspruch 7, wobei der Steuergriff (1732) einen Lenkmechanismus zum Steuern der Ablenkung eines distalen Endes des Katheters (130) aufweist.

9. Anordnung nach Anspruch 8, wobei der Lenkmechanismus eine Lenkeingabevorrichtung aufweist, die mit einem oder mehreren Lenkteilen gekoppelt ist.

10. Anordnung nach Anspruch 1, wobei der Port einen Biopsieport aufweist.

11. Anordnung nach Anspruch 1, ferner mit dem Katheter.

12. Anordnung nach einem der Ansprüche 1 bis 10 und zusätzlich mit mindestens einem der folgenden Merkmale:
dem Katheter (130) mit einem proximalen Ende und einem distalen Ende;
einem Lenkdraht, der am distalen Ende des Katheters (130) angebracht und relativ zum Katheter axial beweglich ist, um zu bewirken, dass das distale Ende des Katheters in mindestens einer Richtung abgelenkt wird;
einem Griffgehäuse, das mit dem proximalen Ende des Katheters an einer Verbindungsschnittstelle funktional verbunden ist, wobei das proximale Ende des mindestens einen Lenkdrahts in das Griffgehäuse einläuft; und
einem ersten Knopf, der durch das Griffgehäuse drehbar gestützt wird.

## Revendications

1. Ensemble de cathéter (1728) pour monter un cathéter (130) sur un dispositif endoscopique (124) comprenant :
un mécanisme de barre de coulissement (1740) qui est fixé sur le dispositif endoscopique (124) et est configuré pour guider l'insertion du cathéter (130) dans le dispositif endoscopique (124) ;
un élément de barre de coulissement allongé (1750) s'étendant vers l'extérieur à partir du dispositif endoscopique (124) et s'étendant parallèlement à un axe central d'un orifice du dispositif endoscopique (124) ; et
une extension latérale (1760) qui, pendant l'utilisation, s'étend entre l'élément de barre de coulissement allongé (1750) et le cathéter (130) pour aligner généralement le cathéter (130) avec l'orifice afin de permettre l'insertion du cathéter (130) dans l'orifice, **caractérisé en ce que** :
l'extension latérale (1760) est configurée pour être acheminée sur l'élément de barre de coulissement allongé (1750) de sorte que l'élément de barre de coulissement allongé (1750) guide l'insertion du cathéter (130) dans l'orifice.

2. Ensemble selon la revendication 1, dans lequel le mécanisme de barre de coulissement (1740) est fixé, de manière permanente, sur le dispositif endoscopique (124).

3. Ensemble selon la revendication 1, dans lequel le mécanisme de barre de coulissement (1740) est fixé, de manière amovible, sur le dispositif endoscopique (124).

4. Ensemble selon la revendication 1, dans lequel le mécanisme de barre de coulissement (1740) comprend un support (1748) configuré pour se raccorder au dispositif endoscopique (124).

5. Ensemble selon la revendication 4, dans lequel le support (1748) est fixé sur l'élément de barre de coulissement allongé (1750).

6. Ensemble selon la revendication 1, dans lequel l'extension latérale (1760) comprend un alésage débouchant configuré pour recevoir l'élément de barre de coulissement allongé (1750).

7. Ensemble selon la revendication 1, dans lequel une poignée de commande (1732) du cathéter (130) comprend l'extension.

8. Ensemble selon la revendication 7, dans lequel une poignée de commande (1732) comprend un mécanisme de direction pour commander la déflexion d'une extrémité distale du cathéter (130).

9. Ensemble selon la revendication 8, dans lequel le mécanisme de direction comprend un dispositif d'entrée de direction couplé à un ou plusieurs éléments de direction.

10. Ensemble selon la revendication 1, dans lequel l'orifice comprend un orifice de biopsie.

11. Ensemble selon la revendication 1, comprenant en outre le cathéter.

12. Ensemble selon l'une quelconque des revendications 1 à 10, et comprenant de plus au moins l'une des caractéristiques suivantes :
le cathéter (130) ayant une extrémité proximale et une extrémité distale ;
un fil de direction fixé sur l'extrémité distale du cathéter (130) et étant axialement mobile par rapport au cathéter pour amener l'extrémité distale du cathéter à dévier dans au moins une direction ;
un logement de poignée raccordé, de manière fonctionnelle, à l'extrémité proximale du cathéter à une interface de connexion, l'extrémité proximale du au moins un fil de direction passant dans le logement de poignée ; et
un premier bouton supporté, en rotation, par le logement de poignée.
